# EUROPEAN PATENT APPLICATION

(11) **EP 2 055 268 A1**
(43) Date of publication of application: **06.05.2009**
(21) Application number: 07790372.2
(22) Date of filing: 20.08.2007
(51) Int. Cl.: A61F 2/28, A61L 27/00

(54) **BONE MODEL, BONE FILLER AND PROCESS FOR PRODUCING BONE FILLER**

(30) Priority: 21.08.2006 JP 2006224785
(71) Applicant: Next21 K.K., Tokyo 113-0033 (JP); The University of Tokyo, Bunkyo-ku, Tokyo 113-8654 (JP)
(72) Inventor: TAKATO, Tsuyoshi, Tokyo 1138654 (JP); SAIJO, Hideto, Tokyo 1138654 (JP); TEI, Yuichi, Tokyo 1138654 (JP); SUZUKI, Shigeki, Tokyo 1130033 (JP); SHIMIZU, Koutaro, Tokyo 1130033 (JP); WASADA, Shinya, Tokyo 1130033 (JP)
(74) Representative: Pitchford, James Edward
(86) International application number: PCT/JP2007/000885
(87) International publication number: WO 2008/023462

(57) **Abstract**

A method for producing a bone filler basically comprising: a bone model producing step (step 1) for producing a bone model; a figure forming material filling step (step 2) for filling a figure forming material into a bone defect site of the bone model obtained in the bone model producing step; and a bone filler producing step (step 3) for producing a bone filler which is to be filled in a bone defect site based on the figure forming material filled in the bone defect site of the bone model in the figure forming material filling step.

## Description

### BACKGROUND OF THE INVENTION

### Field of the Invention

The present invention relates to a bone model, a custom-made bone filler, and a method for producing a custom-made bone filler. In particular, the present invention relates to a bone model whose surface roughness and asymmetry is to be seen clearly by using contour lines, grid patterns (addresses), and the like drawn on the surface thereof. The present invention also relates to a method for producing a bone filler which can precisely produce a bone filler in the following procedures. The shape of a patient's bone is digitally processed by a CT, an MRI, or the like. Based on the digital information, a bone model is formed, and a figure of a bone filler is formed from a figure forming agent by filling or setting the figure forming agents in the bone model. The resultant figure of a bone filler is again digitally processed, and based on the digital data, a bone filler is produced.

### Description of the Related Art

As a medical treatment for a patient who have suffered from a bone loss (patients suffering from a bone defect) in a car accident or the like, an operation is performed to fill the defect site of the bone with an artificial bone in the field of orthopedics, etc. Also, in a surgical treatment, a bone tumor is removed from a lesion site, and the resultant defect site is filled with a bone filler to cure the site. In this treatment, a bone filling agent whose shape is identical to the bone deficit site is required to be obtained.

Japanese Unexamined Patent Application Publication No. 7-284501 discloses the following technique. Three-dimensional data of an insertion site of internal fixation member of an artificial skull and the like (e.g., a defect site) is obtained from tomographic images of X-ray CT or MRI. While showing the three-dimensional image of the insertion site based on the three-dimensional data, the image of internal fixation member is moved on the three-dimensional image, thereby simulating the conformity between the insertion site and the internal fixation member.

Japanese Examined Patent Application Publication No. 6-2137 discloses an apparatus which produces a replica (a model) of an insertion site by obtaining three-dimensional data of an insertion site of internal fixation member of an artificial skull and the like from tomographic images of X-ray CT or MRI, and then by outputting the three-dimensional data by using a cutting device.

Japanese Unexamined Patent Application Publication No. 2003-126124 discloses in claim 1 a bone filling material processing system comprising: a three-dimensional measuring apparatus; and a three-dimensional processing machine connected thereto, wherein bone filling material is processed by the three-dimensional processing machine based on three-dimensional shape data of a bone defect site, the data being obtained by the three-dimensional measuring apparatus.

Japanese Unexamined Patent Application Publication No. 2001-92950 discloses in claim 1 a system for designing an artificial bone for filling a bone defect site based on tomographic images of human bodies, the system comprising: a bone candidate area extracting means, wherein in respective tomographic images photographed at mutually different plural tomographic positions, the pixel area of a predetermined density level is extracted as a bone candidate area; an area selecting means for selecting an area used as a bone area from the bone candidate area (hereinafter referred to as a determined bone area); a bone outline information generating means for generating bone outline information which determines the area to be finally determined as a bone part; a three-dimensional shape data generating means for generating three-dimensional data of the bone defect site based on bone outline information of each tomographic position.

Japanese Patent No. 2,930,420 discloses in claim 1 a method for manufacturing medical device comprising polymer matrix forming step for forming consecutive layers of polymer material by the solid free form fabrication method.

International publication WO05/011536 pamphlet discloses a method for manufacturing an artificial bone by the powder laminating method using RP apparatus.

As above explained, methods for determining a shape of a bone filler are known, in which information on a shape of a bone deficit site is obtained in some way, and the shape of the deficit site is computer simulated, then based on the shape of a simulated deficit site, the shape of the bone deficit site is determined. In these methods, however, a shape of a bone filler is determined based on computer calculation. So it is difficult to incorporate practitioners' know-how, such as doctor's know-how, in the method, although the bone filler produced in these methods is often identical to the shape of the actual defect site. There is also a problem that practitioners have difficulty to image the actual operation, since the shape of the bone filler is determined without their involvement.

Japanese Unexamined Patent Application Publication No. 09-154865 discloses "a bone shape imitating jig for determining a shape of a bone defect site filling material, wherein the jig is formed from a transparent material of a semitransparent material so that the jig is formed on the surface of the bone body part" in claim 1, "a method for determining a shape of bone defect site filling material comprising the steps of putting the shape determining jig on a defect site of a bone body part; selecting the best suited shape determining jig by watching the adjustability of the shape determining jig with the defect part or adjacent shape including the defect part through the transparent shape determining jig" in claim 7, and "a method for determining a shape of bone defect site filling material comprising the steps of: attaching the selected shape determining jig on a proper site covering the defect part; scratching the contour of the defect part on the surface of this shape determining jig; and forming bone defect site filling material for filling the defect part by cutting a corresponding bone defect site filling material based on the scratch of this shape determining jig" in claim 8.

Japanese Unexamined Patent Application Publication No. 09-154865 discloses an invention summarized as follows. A bone filling material plate which fits into the shape of a defect part is obtained by the steps of: putting the shape determining jig of a transparent or semitransparent bone defect site filling material on the defect part; selecting the best suited jig out of several patterns of jigs; estimating the shape of the defect part through the best suited transparent jig; and scratching (paragraph [0013]) and cutting the jig (paragraph [0014]).

In this method, practitioner's know-how is highly likely to be incorporated in the bone filler. But in this method, the shape of a defect site is assumed by putting transparent jig to the deficit site. Thus, whether the shape of the bone filler fits the defect site or not depends on the practitioner's experiences. Therefore, there is a problem that a suitably-shaped bone filler cannot always be obtained.

Also, there is a problem that a patient of bone deformity has the deformed bone shape of a specific part, thereby causing asymmetry of bone structures. In order to recover the symmetry, a bone filler is put in a defect site of a bone. However it was difficult to produce a bone filler having a proper shape.

Also, in the field of conventional plastic surgery and cosmetic surgery, a desired bone structure have been achieved by cutting bones, and a facial shape have been reshaped by putting silicon, cellulite, or the like in specific parts. However, there are the following problems. When foreign material such as silicon is embedded in a body, a large amount of immunosuppressive drug is required. And since it is foreign material to a living body forever, the patient must undergo routine medical examination. Furthermore, the patient may suffer from inflammation because silicon and the like do not conform to the human body.

Since bone filler is generally fragile, there is a problem that it can be broken when strong impact or strong force is added thereto from outside. There is another problem that the bone filler which was once filled in a bone defect site may be detached when a force is added thereto from outside, because the bone filler is not actual bone tissues of a patient.

As for a patient of bone deformity, the deformation of bones can be appreciated by actually watching the affected area. But there is a problem that objective information on the level of the bone deformity is difficult to be obtained. In the medical treatment of bone deformity, in the medical treatment of plastic surgery, or in the medical treatment of cosmetic surgery, there is another problem that the variation of the physical appearance (e.g. rise and fall of the surface of a face) between before and after the medical treatment cannot be seen objectively.

The object of the present invention is to provide a bone model by which bone deformity of a patient and the like can be appreciated.

The object of the present invention is to provide a method for producing a bone filler which can be filled in a bone defect site with accuracy.

The object of the present invention is to provide a method for producing a bone filler which can correct bone deformity (e.g., asymmetry of bone) effectively.

The object of the present invention is to provide a method for producing a cast having a proper shape, which protects a bone filler from outer impacts as well as reflects the shape of the bone filler.

The object of the present invention is to provide an appearance model which can show the deformation degree of the physical appearance of a specific part of a patient suffering from bone deformity objectively. The preset invention is also aimed at providing an appearance model which can show the variation of the physical appearance between before and after the medical treatment in a surgical operation and the like, and a method for providing the model.

Epitheses have been made based on the experiences of producers. So the quality of an epithesis obtained varied based on the skill of the producers. There is also a case that asymmetry epithesis, which are not suitable in terms of physical appearance, are obtained. Furthermore, for example, when an epithesis of a specific part of a face is made, impression material is used on the site to take a moulage of the site. So there was a problem that the patients feel painful. Therefore, the object of the present invention is to provide a method for producing a symmetrical epithesis which is less painful for patients, or to provide a method for producing a mold for producing the epitheses.

### SUMMARY OF THE INVENTION

When a bone filler is produced totally based on analog information which is based on the experience of a practitioner, the accuracy of the obtained bone filler varies largely based on the skill of the practitioner. On the other hand, when a bone filler is produced totally based on degital information, the experience of the practitioner cannnot be reflected on the obtained bone filler. Thus, the present invention is based on the idea that a quite accurate bone filler can be produced by using degital information in a process in which degital imformation is preferred to be used, and also by reflecting analog information of a practitiner's knowledge while adjusting environment in which the practitiner's knowledge is easy to be reflected.

The present invention is particulary based on the idea that deformation or asymmetry of a patient's bone can be grasped by contour lines or grid patterns drawn on the surface of the patient's bone model. The present invention is further based on the idea that a bone filler having a proper shape can be obtained based on the figure-forming material which make up for the grasped deformation.

The present invention relates to a method for producing a bone filler which can produce accurate bone filler basically comprising the steps of: producing a custom-made patient's bone model; producing a figure of a bone filler by filling figure-forming material in a defect site by using the bone model; and producing a bone filler based on the figure of a bone filler.

The present invention also relates to the idea of obtaining a bone filler having a proper shape by the steps of: digitizing a patient's bone by CT scans; producing a bone model whereon contour lines, grid patterns, or the like is drawn based on the digitized information; grasping the deformation of a patient's bone based on the lines drawn on the model; producing a figure of a bone filler by filling or setting figure-forming material so that the deformation is corrected; redigitizing the obtained figure; and producing a bone filler based on the redigitized information.

The first aspect of the present invention relates to a method for producing a one filler comprising: a digital information of bones obtaining step comprising: a step of photographing a specific part of a patient; and a step of obtaining digital information of bones, the digital information including cross-sectional view of pluralities of the bones, the bones located at the specific part of the patient; a bone model producing step for producing a bone model based on the digital information, the bone model being located at the specific part of the patient, the digital information including cross-sectional view of pluralities of the bones, the digital information being obtained in the digital information of bones obtaining step; a figure-forming material setting step for setting figure-forming material on the bone model produced in the bone model producing step; a figure-forming material digital information obtaining step for obtaining digital information of figure-forming material by photographing the bone model, the bone model on which the figure-forming material being set in the figure-forming material setting step; and a bone filler producing step for producing a bone filler based on the digital information of figure-forming material, the digital information being obtained in the figure-forming material digital information obtaining step. Namely, steps which are preferred to be performed digitally, such as model forming, bone filler producing after figure-forming, and the like are preformed digitally. On the other hand, as described below, an environment where a practitioner's analog skill can be exerted is developed, for example by drawing contour lines on the surface of the bone model, and then the practitioner reflects his analog skill by setting figure-forming material on the bone model based on the contour lines. In this way, highly qualified bone filler can be produced.

A preferred embodiment of the first aspect of the present invention is one of the above described method for producing a bone filler wherein the step of photographing a specific part of a patient is a step for obtaining digital information of a bone including cross-sectional view of pluralities of the bones by a CT scan or an MRI, the bones being located at the specific part of the patient, and wherein the figure-forming material digital information obtaining step is a step for obtaining the digital information of figure-forming material by a CT scan or an MRI. Namely, pluralities of cross-sectional views of a figure including a bone or figure-forming material can be easily obtained by a CT scan or an MRI. And a three-dimensional digital information of the bone or the figure-forming material can be easily obtained by a computer and the like based on the figures photographed by the CT scan or the MRI. A preferred embodiment of the first aspect of the present invention relates to one of the above described bone filler wherein the specific part of the patient is a site including one of a patient's skull, a lower jaw, an upper jaw, four limbs, or a pelvis. Since these sites include symmetrical parts, deformations can be easily grasped.

A preferred embodiment of the first aspect of the present invention relates to one of the above described method for producing a bone filler wherein the bone model produced in the bone model producing step has contour lines or grid patterns drawn on the surface thereof.If contour lines or grid patterns are drawn on the surface thereof, the practitioner can grasp a bone deformation, a depressed area, and the like quite easily based on the obtained bone model. As a result, quite accurate bone filler can be obtined.

A preferred embodiment of the first aspect of the present invention relates to one of the above described method for producing a bone filler wherein the bone model produced in the bone model producing step is a bone model containing gypsum. A preferred embodiment of the first aspect of the present invention relates to one of the above described method for producing a bone filler wherein the bone model producing step is a step for producing a bone model by the rapid prototype method, the injection molding method, the laminate molding method by cutting, or a molding method using processing equipment having a machining center. If a bone model mainly contains gypsum, a bone model can easily be produced by: the rapid prototype method; the injection molding method for producing a bone model using a mold which is designed based on the bone digital information photographed by a CT scan, an MRI, or the like; a molding method using a processing equipment having a machining center based on the obtained bone digital information; a molding method using an NC controllable cutting equipment having a multiple-spindle drilling machine based on the obtained bone digital information.

A preferred embodiment of the first aspect of the present invention relates to one of the above described method for producing a bone filler wherein the bone model produced in the bone model producing step contains calcium-based material and polyvinyl alcohol resin, and wherein the polyvinyl alcohol resin is 2 to 8 weight parts when the total weight of the calcium-based material and the polyvinyl alcohol resin is 100 weight parts.This bone model can be obtained in particular by the injection molding quite quickly and highly accurately. As a result, bone filler can be produced with accurate. A preferred embodiment of the first aspect of the present invention relates to one of the above described method for producing a bone filler wherein the bone model produced in the bone model producing step is made of material containing α-type hemihydrate gypsum and polyvinyl alcohol resin, and wherein the polyvinyl alcohol resin is 2 to 8 weight parts when the total weight of the calcium-based material and the polyvinyl alcohol resin is 100 weight parts.

A preferred embodiment of the first aspect of the present invention relates to one of the above described method for producing a bone filler wherein the bone model contains gypsum as the main ingredient, and wherein the figure-forming material contains wax or plastic more than 90 weight parts per 100 weight parts of the total amount. A preferred embodiment of the first aspect of the present invention relates to one of the above described method for producing a bone filler wherein the bone model contains gypsum as the main ingredient, and wherein the figure-forming material contains wax more than 90 weight parts per 100 weight parts of the total amount. A preferred embodiment of the first aspect of the present invention relates to one of the above described method for producing a bone filler wherein the bone model contains gypsum as the main ingredient, wherein the figure-forming material contains wax more than 90 weight parts per 100 weight parts of the total amount, and wherein the figure-forming material contains titanium oxide of the rutile type of 2 to 5 weight parts per 100 weight parts of the total amount. Namely, when the bone model having this composition and figure-forming material are photographed by a CT scan or an MRI, a bone model portion and a figure-forming material portion can be precisely analyzed, thereby producing a bone filler with high accuracy.

A preferred embodiment of the first aspect of the present invention relates to one of the above described method for producing a bone filler wherein the bone filler producing step is a step for producing a bone filler by the rapid prototype method. A custom-made bone filler can be produced quickly and accurately by the Rapid Prototype Method.

A preferred embodiment of the first aspect of the present invention relates to one of the above described method for producing a bone filler wherein the bone filler obtained in the bone filler producing step is produced from one or more than one of hydroxyapatite, carbonate apatite, fluorapatite, chlorapatite, β-TCP, α-TCP, calcium metaphosphate, tetra-calcium phosphate, octa-calcium phosphate, calcium hydrogen phosphate, calcium dihydrogen phosphate, calcium pyrophosphate, salts thereof, or solvates thereof. For example, when a bone filler is produced from these materials by the injection molding method, phase change occurs at the time of molding, turning it into a bone filler having preferred characteristics.

The second aspect of the present invention relates to a bone model whereon contour lines or grid patterns are drawn. Since these contour lines or grid patterns are drawn on the surface, the deformation of a bone model can easily be grasped. A preferred embodiment of this aspect relates to one of the above described bone model which is a reproduction of a bone shape of a patient's specific part. Also, another preferred embodiment relates to the above described bone model which is a reproduction of a bone shape of a patient's skull. Namely, if a custom-made bone model of a specific patient is used, deformation of the patient's bone can be precisely grasped. As a result, the bone model is effective to grasp the shape of a bone filler properly, and a bone filler having a proper shape can be obtained.

The third aspect of the present invention relates to a method for producing a bone filler comprising: a bone model producing step for producing a bone model; a figure-forming material filling step for filling figure-forming material in a bone defect site of the bone model, the bone model being obtained in the bone model producing step; and a bone filler producing step for producing a bone filler, the bone filler being filled in the bone defect site based on the figure-forming material, the figure forming material being filled in the bone defect site of the bone model in the figure-forming material filling step.

A preferred embodiment of the third aspect of the present invention relates to one of the above described method for producing a bone filler, wherein the bone model producing step is a step for producing a bone model by the rapid prototype method. A patient's bone model having a partial defect, for example, can be quickly and precisely produced by the rapid prototype method. A preferred embodiment of the third aspect of the present invention relates to one of the above described method for producing a bone filler the bone model produced in the bone model producing step has contour lines or grid patterns drawn on the surface thereof. Namely, since the deformation of a patient's bone can be grasped properly based on the bone model, a proper bone filler can be produced for patients.

A preferred embodiment of the third aspect of the present invention relates to one of the above described method for producing a bone filler, wherein the figure-forming material used in the figure-forming material filling step differs from the bone model in one of the X-ray permeability, the infrared rayspermeability, or the ultraviolet ray permeability. Since the figure-forming material differs from the bone model in one of the X-ray permeability, the infrared rays permeability, or the ultraviolet rays permeability, the shape of figure-forming material can be analyzed with figure-forming material put on the bone model. In this way, the figure-forming material can be prevented from being deformed when it is taken out of the bone model, and the figure-forming material can be prevented from being left partially in the bone model. As a result, the shape of the bone defect site can be precisely grasped.

A preferred embodiment of the third aspect of the present invention relates to one of the above described method for producing a bone filler, wherein the figure-forming material used in the figure-forming material filling step contains titanium oxide of the rutile type of 2 to 5 weight parts per 100 weight parts of the total amount. The figure-forming material and the bone model can be distinguished from each other by an X-ray CT and the like by containing titanium oxide of the rutile type in the figure-forming material.

A preferred embodiment of the third aspect of the present invention relates to one of the above described method for producing a bone filler, wherein the bone model produced in the bone model producing step contains gypsum as the main ingredient, wherein the figure-forming material used in the figure-forming material filling step contains wax more than 90 weight parts per 100 weight parts of the total amount, and wherein the figure-forming material contains titanium oxide of the rutile type of 2 to 5 weight parts per 100 weight parts of the total amount. The figure-forming material and the bone model can be distinguished from each other by an X-ray CT and the like by containing titanium oxide of the rutile type in the figure-forming material.

A preferred embodiment of the third aspect of the present invention relates to one of the above described method for producing a bone filler, wherein the bone filler producing step is a step for producing a bone filler by the rapid prototype method. Namely, information on the shape of the bone deficit site can be obtained by an X-ray CT and the like, the bone filler can be produced rapidly and precisely by the rapid prototype method.

A preferred embodiment of the third aspect of the present invention relates to one of the above described method for producing a bone filler, wherein the bone filler producing step comprises: a kneading step for kneading ingredient comprising calcium-based material and material comprising binder; a molding step for obtaining a molded body having a predetermined shape from a kneaded material with an injection molding machine having a mold, the kneaded material being obtained in the kneading step; a binder removal step for obtaining a degreased body by removing the binder contained in the molded body obtained in the molding step; and a sintering step for obtaining a sintered body by heating and sintering the degreased body obtained in the binder removal step. Information on the shape of the bone deficit site can be obtained by an X-ray CT and the like, and a mold can be made based on this shape information. And then a bone filler having a precise shape can be produced.

A preferred embodiment of the third aspect of the present invention relates to one of the above described method for producing a bone filler, wherein further comprising a step of penetrating or administering an osteogenesis/chondrogenesis promoter, a joint disease therapeutic agent, a preventive and/or therapeutic agent for bone/cartilage disease, a bone-regenerating agent, a bone resorption-suppressing substance, an angiogenesis promoter, an antibacterial agent, an antibiotics, or an anticancer agent into the bone filler, the bone filler beeing obtained in the bone filler producing step. By impregnating or coating a predetermined pharmaceutical agent on the bone filler, a bone filler having various pharmaceutical effect can be provided.

The fourth aspect of the present invention relates to a method for producing a bone filler comprising: a bone model producing step for producing a bone model; a figure-forming material setting step for setting figure-forming material on the bone model, the bone model being obtained in the bone model producing step; and a bone filler producing step for producing a bone filler based on the figure-forming material, the figure-forming material being set on the bone model in the figure-forming material setting step.Note that the figure forming material setting step is preferably a step for setting figure forming material so as to correct asymmetry of a bone model by using a bone model obtained in the bone model producing step. In this way, a bone filler which can correct deformation of a bone can be obtained.

A preferred embodiment of the fourth aspect of the present invention relates to a method for producing one of the above described bone filler, wherein the bone model produced in the bone model producing step has contour lines or grid patterns drawn on the surface thereof.

A preferred embodiment of the fourth aspect of the present invention relates to a method for producing one of the above described bone filler, wherein the figure-forming material used in the figure-forming material filling step differs from the bone model in one of the X-ray permeability, the infrared ray permeability, or the ultraviolet ray permeability. A preferred embodiment of the fourth aspect of the present invention relates to a method for producing one of the above described bone filler, wherein the bone model is a bone model of a patient having bone defect, a patient suffering from bone deformation, or a patient of cosmetic surgery.

The fifth aspect of the present invention relates to a method for producing a bone filler and a cast comprising: a bone digital information obtaining step for obtaining bone digital information including a cross-sectional view of pluralities of the bones, the bones located at the specific part of the patient, by photographing the specific part of the patient; a bone model producing step for producing a bone model based on the digital information, the bone model being located at the specific part of the patient, the digital information including a cross-sectional view of pluralities of the bones, the digital information being obtained in the digital information of bones obtaining step; a figure-forming material setting step for setting figure-forming material on the bone model, the figure-forming material being used for a bone filler, the bone model produced in the bone model producing step, and for setting figure-forming material on the bone model, the figure-forming material being used for cast forming, the figure-forming material including material which is different from the figure-forming material used for a bone filler; a figure-forming material digital information obtaining step for obtaining digital information of figure-forming material by photographing the bone model, the bone model whereon the figure-forming material is set in the figure-forming material setting step; and a bone filler and a cast producing step for producing a bone filler and a cast based on the digital information of the figure-forming material, the figure-forming material obtained in the figure-forming material digital information obtaining step.

According to the method for producing a bone filler and a cast of this aspect, a preferred bone filler can be obtained, and a cast which can support the bone filler properly can be designed. The figure forming material used for forming a cast contains material which is different from the figure forming material used for a bone filler. So these shapes can be distinguished from each other by photographing them with a CT scan, an MRI, and the like.

The fifth aspect of the present invention relates to a method for producing a cast comprising: a bone and soft tissues digital information obtaining step for obtaining bone digital information including cross-sectional view of pluralities of the bones, the bones located at the specific part of the patient, and digital information on the soft tissues, the soft tissues located around the bone, by photographing the specific part of the patient; a cast producing step for producing a cast of the specific part of the patient based on the digital information, the digital information including a cross-sectional view of pluralities of bones and soft tissues obtained in the bone and soft tissue digital information obtaining step. Since a cast is produced based on the digital information of soft tissues, a custom-made cast having a shape which is suitable for patients can be produced. Note that as a method for producing a bone filler or a cast of the present invention, or as a method for producing a cast, a preferred embodiment or configuration of the above described method for producing a bone filler can be adopted as appropriate.

The sixth aspect of the present invention relates to an appearance model of a specific part of a body whereon contour lines or grid patterns are drawn. Since contour lines or grid patterns are drawn on the surface thereof, a deformation of a specific part can objectively be grasped. In particular, the degree of deformation can be grasped objectively by comparing an appearance model before and after the operation.

The sixth aspect of the present invention relates to the appearance model is a reproduction of the surface of a specific part of a patient's body. The specific parts of a patient, for example, are the above described parts. They are, specifically, a face, a head, four limbs, a chest, a lower abdomen, a waist, and the like.

The sixth aspect of the present invention relates to a cross-sectional view digital information obtaining step for obtaining digital information on a cross-sectional view of a specific part of a patient, the specific part including a cross-sectional view of pluralities of bones and soft tissues of the specific part of the patient, by photographing the specific part; a drawing information obtaining step for calculating the height of each part of the surface on the specific part from a base level based on the digital information, the digital information including a cross-sectional view of pluralities of bones and soft tissues obtained in the cross-sectional view digital information obtaining step, or a drawing information obtaining step for calculating the distortion on the surface of each part of the surface of the specific part from a base level; an appearance model producing step for producing a surface model on the specific part of the patient by the rapid prototype method, as well as drawing contour lines or grid patterns based on the distortion on the surface or height obtained in the drawing information obtaining step. In this producing method, an appearance model can be properly produced. Since appearance models before and after the operation can be obtained, the degree of change in appearance before and after the operation can be shown in a surgical operation and the like.

The seventh aspect of the present invention relates to a method for producing an epithesis comprising: a cross-sectional view digital information obtaining step for obtaining digital information on a cross-sectional view of a specific part, the cross-sectional view including a cross-sectional view of pluralities of bones and soft tissues of the specific part of a patient, by photographing the specific part of the patient, and a three-dimensional digital figure obtaining step for obtaining a three-dimensional digital figure of the specific part based on the digital information, the digital information including the cross-sectional view of pluralities of bones and soft tissues obtained in the cross-sectional view digital information obtaining step, an epithesis figure data obtaining step for obtaining epithesis figure data based on the three-dimensional digital figure of the specific part obtained in the three-dimensional digital figure obtaining step; and an epithesis producing step by the rapid prototype method based on epithesis figure data obtained in the epithesis figure data obtaining step.

A preferred embodiment of the seventh aspect of the present invention relates to a method for producing a mold used for producing an epithesis, the method comprising: a cross-sectional view digital information obtaining step for obtaining digital information on a cross-sectional view of a specific part, the cross-sectional view including a cross-sectional view of pluralities of bones and soft tissues of the specific part of a patient, by photographing the specific part of the patient, and a three-dimensional digital figure obtaining step for obtaining a three-dimensional digital figure of the specific part based on the digital information, the digital information including the cross-sectional view of pluralities of bones and soft tissues obtained in the cross-sectional view digital information obtaining step, an appearance model producing step for producing an appearance model of the specific part based on the three-dimensional digital figure of the specific part obtained in the three-dimensional digital figure obtaining step; a figure forming material setting step for setting figure forming material on the appearance model, the appearance model obtained in the appearance model producing step;a figure forming material digital information obtaining step for obtaining figure forming material digital information by photographing a bone model on which a figure forming material is set in the figure forming material setting step; a mold information obtaining step for obtaining digital data on a mold for producing an epithesis figure based on the digital information of the figure forming material obtained in the figure forming material digital information obtaining step; and a mold producing step for producing a mold obtained in the mold information obtaining step. For example, when a part having symmetrical appearance is partially defected, the defected part can be reshaped based on the remaining part. Therefore, a method for producing a symmetrical epithesis, or a method for producing a mold which is used for producing an epithesis can be provided. In the present invention, shape information of a specific part is obtained by a CT scan and the like, and an epithesis is designed by a computer based on the information. Since impression material is not needed to be put directly on a patient, a method for producing a minimally invasive epithesis, or a method for producing a mold which is used for producing a minimally invasive epithesis can be provided.

In the present invention, since contour lines or grid patterns are drawn on the surface of the bone model, a bone model which can show the deformation of patient's bones can be provided.

In the present invention, since a bone filler is designed based on a patient's bone model, a method for producing a bone filler which can precisely produce a bone filler to be filled in a bone deficit site can be provided. In particular, when a patient's bone model whereon contour lines or grid patterns are drawn is used, the deficit site or the deformation of the patient's bone can be grasped properly. Therefore, a bone filler which is to be filled in the bone deficit site can be produced with accuracy.

In the present invention, since a bone filler is designed based on a patient's bone model, a method for producing a bone filler which can effectively correct bone deformation (bone asymmetry) can be provided. In particular, when a patient's bone model whereon contour lines or grid patterns are drawn is used, the deformation of the patient's bone can be grasped properly. Therefore, a bone filler which is to be filled in the bone deficit site can be produced with accuracy.

The present invention can provide a method for producing a cast having a proper shape, which protects a bone filler from outer impacts as well as reflects the shape of the bone filler.

The the present invention can provide an appearance model which can show the deformation degree of a specific part of a patient's deformed bone objectively. In the present invention, appearance models which are before and after the medical treatment can be obtained. Thus, the preset invention can provide a model which can show the variation of the physical appearance before and after the medical treatment in a surgical operation and the like, and a method for producing the model.

In the present invention, for example, when a part having symmetrical appearance is partially defected, the defected part can be reshaped based on the remaining part. Therefore, a method for producing a symmetrical epithesis, or a method for producing a mold which is used for producing an epithesis can be provided. In the present invention, shape information of a specific part is obtained by a CT scan and the like, and an epithesis is designed by a computer based on the information. Since impression material is not needed to be put directly on a patient, a method for producing a minimally invasive epithesis, or a method for producing a mold which is used for producing a minimally invasive epithesis can be provided.

### BREIF DESCRIPTION OF THE DRAWINGS

Fig. 1 is an example of a flow showing basic steps of the method for producing a bone filler of the present invention.
Fig. 2 is a schematic view showing each step of the present example.
Fig. 3 is a CT image, in place of a diagram. Fig. 3(a) is a CT image of cheek parts. Fig. 3(b) is a CT image of a lower jaw part.
Fig. 4 shows photographs and three-dimensional figures, in place of a diagram, of bone models obtained in the example. Fig. 4(a) shows a gypsum model (a bone model) obtained. Fig. 4(b) shows a side view of a bone model. Fig. 4(c) shows a design drawing of a bone model whereon contour lines are drawn.
Fig. 5 shows photographs, in place of a diagram, of a bone model whereon figure forming material is set. Fig. 5(a) is a front view. Fig. 5(b) is a side view. Fig. 5(c) is a bottom view.
Fig. 6 shows CT images, in place of a diagram, of a bone model whereon figure forming material is set. Fig. 6(a) is a CT image of cheek parts. Fig. 6(b) is a CT image of a lower jaw part.
Fig. 7 shows photographs, in place of a diagram, of a bone filler obtained in the present example. Fig. 7(a) shows a bone filler filled in cheek parts. Fig. 7(b) shows the other side of the bone filler. Fig. 7(c) shows a bone filler filled in a lower jaw part. Fig. 7(d) shows the other side of the bone filler.
Fig. 8 shows photographs, in place of a diagram, of a gypsum figure (an appearance model) whereon contour lines, which show unevenness of a patient's face treated with a bone filler obtained in the example, are drawn. Fig. 8(a) shows a photograph before the treatment. Fig. 8(b) shows a photograph after the treatment.

### Reference Numeral

### DESCRIPTION OF THE PREFFERED EMBODIMENTS

### 1. The Method for Producing a Bone Filler

The first aspect of the present invention relates to a method for producing a bone filler comprising: a digital information of bones obtaining step comprising: a step of photographing a specific part of a patient; and a step of obtaining digital information of bones, the digital information including cross-sectional view of pluralities of the bones, the bones located at the specific part of the patient; a bone model producing step for producing a bone model based on the digital information, the bone model being located at the specific part of the patient, the digital information including cross-sectional view of pluralities of the bones, the digital information being obtained in the digital information of bones obtaining step; a figure-forming material setting step for setting figure-forming material on the bone model produced in the bone model producing step; a figure-forming material digital information obtaining step for obtaining digital information of figure-forming material by photographing the bone model, the bone model on which the figure-forming material being set in the figure-forming material setting step; and a bone filler producing step for producing a bone filler based on the digital information of figure-forming material, the digital information being obtained in the figure-forming material digital information obtaining step.Namely, steps which are preferred to be performed digitally, such as model forming, bone filler producing after figure-forming, and the like are preformed digitally On the other hand, as described below, an environment where a practitioner's analog skill can be exerted is developed, for example by drawing contour lines on the surface of the bone model, and then the practitioner reflects his analog skill by setting figure-forming material on the bone model based on the contour lines. In this way, highly qualified bone filler can be produced.

A preferred embodiment of the first aspect of the present invention is one of the above described method for producing a bone filler wherein the step of photographing a specific part of a patient is a step for obtaining digital information of a bone including cross-sectional view of pluralities of the bones by a CT scan or an MRI, the bones being located at the specific part of the patient, and wherein the figure-forming material digital information obtaining step is a step for obtaining the digital information of figure-forming material by a CT scan or an MRI. Namely, pluralities of cross-sectional views of a figure including a bone or figure-forming material can be easily obtained by a CT scan or an MRI. And a three-dimensional digital information of the bone or the figure-forming material can be easily obtained by a computer and the like based on the figures photographed by the CT scan or the MRI. A preferred embodiment of the first aspect of the present invention relates to one of the above described bone filler wherein the specific part of the patient is a site including one of a patient's skull, a lower jaw, an upper jaw, four limbs, or a pelvis. Since these sites include symmetrical parts, deformations can be easily grasped. As a CT imager used for a CT scan and an MRI, a known imager can be used as appropriate. Note that the CT imager is preferred to be connected with a computer. And the computer preferably comprises: an input and output device connected to a CT device and a monitor; a memory for storing image data obtained by a CT or an MRI; a controller (a computing part) for performing arithmetic computation; a main memory storing a program for obtaining three-dimensional digital data of the photographed object part based on pluralities of CT image data or MRI image data; and busses connecting each device together.

A preferred embodiment of the first aspect of the present invention relates to one of the above described method for producing a bone filler wherein the bone model produced in the bone model producing step has contour lines or grid patterns drawn on the surface thereof. If contour lines or grid patterns are drawn on the surface thereof, the practitioner can grasp a bone deformation, a depressed area, and the like quite easily based on the obtained bone model. As a result, quite accurate bone filler can be obtined.

A preferred embodiment of the first aspect of the present invention relates to one of the above described method for producing a bone filler wherein the bone model produced in the bone model producing step is a bone model containing gypsum. A preferred embodiment of the first aspect of the present invention relates to one of the above described method for producing a bone filler wherein the bone model producing step is a step for producing a bone model by the rapid prototype method, the injection molding method, the laminate molding method by cutting, or a molding method using processing equipment having a machining center. If a bone model mainly contains gypsum, a bone model can easily be produced by: the rapid prototype method; the injection molding method for producing a bone model using a mold which is designed based on the bone digital information photographed by a CT scan, an MRI, or the like; a molding method using a processing equipment having a machining center based on the obtained bone digital information; a molding method using an NC controllable cutting equipment having a multiple-spindle drilling machine based on the obtained bone digital information. The laminate molding method by cutting is, for example, performed in the way disclosed in JP-A 08-290347 that is "a laminate molding method, wherein a three-dimensional shape is divided into several layers, then each layer is formed and laminated sequentially, comprising the steps of: casting a plate material into three-dimensional curved shape by tools such as a cutting tool or a grinding tool based on the instruction of a three-dimensional processing program which is made for each layer based on three-dimensional numerical data; laminating unprocessed plate material on this processed plate material; and then repeating the step of casting an unprocessed plate material into three-dimensional curved shape by the above tools". Or it may be a laminate molding method for laminating three-dimensional shaped object into two-dimensional half shape by cutting sheet shaped material based on two-dimensional shaped data of each layer of the object, then sequentially laminating the sheet, which is disclosed in JP-A 03-244510 and JP-A 05-313584. Or it may be a laminate molding method for laminating three-dimensional shaped object into two-dimensional half shape by irradiating light beam on the surface of liquid light hardening resin, forming a hardened layer having a predetermined shape, further providing unhardened liquid light hardening resin on this hardened layer and again irradiating light beam, repeating this process and laminating a new layer on the hardened layer. Also, as a machining center, a well known machining center can be used as appropriate. For example, a machining center disclosed in JP-A 2004-074376, JP-A 2003-94264, or JP-A 2001-150262 can be used as appropriate. Also, as a multi spindle drilling machine (e.g., five axis spindle drilling machine) the ones disclosed in JP-A 2006-5257, JP-A 2001-230223, or JP-A 2000-176715 can be used as appropriate.

A preferred embodiment of the first aspect of the present invention relates to one of the above described method for producing a bone filler wherein the bone model produced in the bone model producing step contains calcium-based material and polyvinyl alcohol resin, and wherein the polyvinyl alcohol resin is 2 to 8 weight parts when the total weight of the calcium-based material and the polyvinyl alcohol resin is 100 weight parts. This bone model can be obtained in particular by the injection molding quite quickly and highly accurately. As a result, bone filler can be produced with accurate. A preferred embodiment of the first aspect of the present invention relates to one of the above described method for producing a bone filler wherein the bone model produced in the bone model producing step is made of material containing α-type hemihydrate gypsum and polyvinyl alcohol resin, and wherein the polyvinyl alcohol resin is 2 to 8 weight parts when the total weight of the calcium-based material and the polyvinyl alcohol resin is 100 weight parts.

A preferred embodiment of the first aspect of the present invention relates to one of the above described method for producing a bone filler wherein the bone model contains gypsum as the main ingredient, and wherein the figure-forming material contains wax or plastic more than 90 weight parts per 100 weight parts of the total amount. A preferred embodiment of the first aspect of the present invention relates to one of the above described method for producing a bone filler wherein the bone model contains gypsum as the main ingredient, and wherein the figure-forming material contains wax more than 90 weight parts per 100 weight parts of the total amount. A preferred embodiment of the first aspect of the present invention relates to one of the above described method for producing a bone filler wherein the bone model contains gypsum as the main ingredient, wherein the figure-forming material contains wax more than 90 weight parts per 100 weight parts of the total amount, and wherein the figure-forming material contains titanium oxide of the rutile type of 2 to 5 weight parts per 100 weight parts of the total amount. Namely, when the bone model having this composition and figure-forming material are photographed by a CT scan or an MRI, a bone model portion and a figure-forming material portion can be precisely analyzed, thereby producing a bone filler with high accuracy.

A preferred embodiment of the first aspect of the present invention relates to one of the above described method for producing a bone filler wherein the bone filler producing step is a step for producing a bone filler by the rapid prototype method. A custom-made bone filler can be produced quickly and accurately by the Rapid Prototype Method.

A preferred embodiment of the first aspect of the present invention relates to one of the above described method for producing a bone filler wherein the bone filler obtained in the bone filler producing step is produced from one or more than one of hydroxyapatite, carbonate apatite, fluorapatite, chlorapatite, β-TCP, α-TCP, calcium metaphosphate, tetra-calcium phosphate, octa-calcium phosphate, calcium hydrogen phosphate, calcium dihydrogen phosphate, calcium pyrophosphate, salts thereof, or solvates thereof. For example, when a bone filler is produced from these materials by the injection molding method, phase change occurs at the time of molding, turning it into a bone filler having preferred characteristics.

### 2 Bone Model

The second aspect of the present invention relates to a bone model whereon contour lines or grid patterns are drawn. Since these contour lines or grid patterns are drawn on the surface, the deformation of a bone model can easily be grasped. The contour lines are drawn, for example, at every height of 0.1 mm to 1 cm, preferably at every height of 0.5 mm to 5 mm, further preferably at every height of 0.5 mm to 2 mm, in which the heights are measured from a certain point of a bone model (the lowest point). If the bone model is that of a skull, for example, contour lines are preferred to be drawn based on the back of the head. The part on which contour lines are drawn may be all over the bone model or may be a part of the bone model. Also, the bone model of a skull may be a model of all over the skull, or may be a bone model of a necessary part. The necessary part may be a front half of the skull (a mask part). When a bone filler used inside the mouth, the necessary part may be only part of a lower jaw, an upper jaw, or both lower and upper jaw. The grid patterns drawn on the surface of a bone model is not specifically limited if they are lattice-shaped. Only grid points may be drawn on the surface thereof. The patterns may be drawn, for example, at every 0.1 mm to 3 cm, preferably at every 0.5 mm to 1 cm, further preferably at every 1 mm to 5 mm. The patterns may be drawn all over or a part of the bone model. This bone model can be produced by a known method, for example, the rapid prototype method described below. In particular, the method is as follows. When a bone model is produced by the rapid prototype method, three-dimensional information of a bone model is obtained based on pluralities of digital images. Since information on the height of the surface of a bone model can be obtained based on the three-dimensional information, drawing information on the site where contour lines are drawn based on the obtained height information is obtained, and the contour lines may be drawn based on the obtained drawing information when the bone model is produced by the rapid prototype method in which ink can be sprayed on the site where a computer assigned. Also, when grid patterns are drawn, for example, when a bone model is produced by the rapid prototype method, three-dimensional information of a bone model is obtained based on pluralities of digital images. Since information on two-dimensional site of the surface of a bone model can be obtained based on the three-dimensional information, drawing information on the site where grid patterns are drawn based on the obtained two-dimensional site information is obtained, and the grid patterns may be drawn based on the obtained drawing information when the bone model is produced by the rapid prototype method in which ink can be sprayed on the site where a computer assigned.

A preferred embodiment of this aspect relates to the above described bone model which is a reproduction of a bone shape of a patient's specific part. Also, another preferred embodiment relates to the above described bone model which is a reproduction of a bone shape of a patient's skull. Namely, if a custom-made bone model of a specific patient is used, deformation of the patient's bone can be precisely grasped. As a result, the bone model is effective to grasp the shape of a bone filler properly, and a bone filler having a proper shape can be obtained.

### 3 Method for Producing Bone Filler

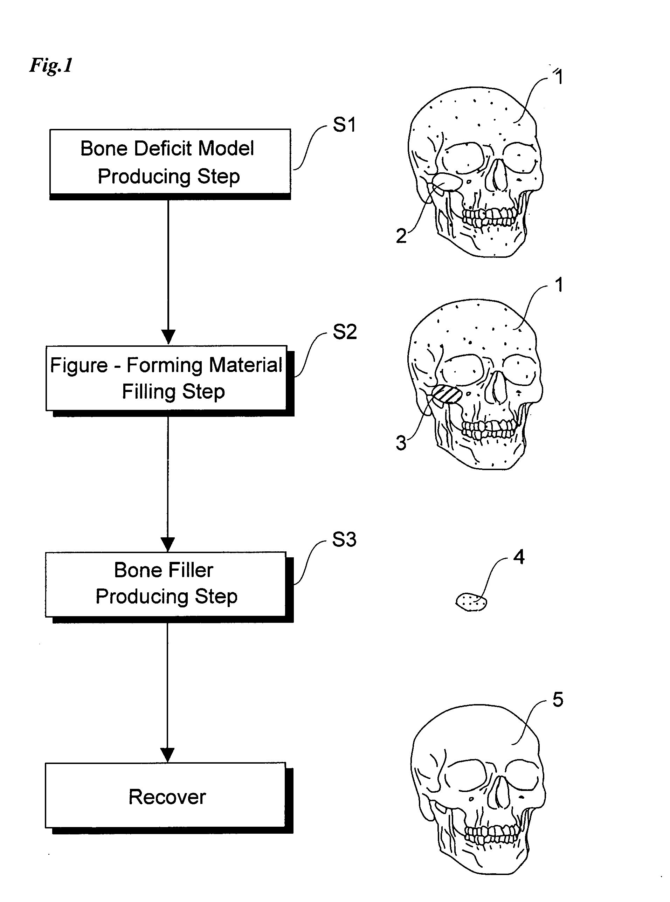

Hereinafter, a method for producing a bone filler is explained according to a figure. Fig. 1 is an example of a flow showing basic steps of the method for producing a bone filler of the present invention. In the figure, the reference symbol "S" means step. As shown in Fig. 1, the method for producing a bone filler of the present invention basically comprises: a bone model producing step (step 1) for producing a bone model (1); a figure-forming material filling step (step 2) for filling figure-forming material (3) into a bone defect site (2) of the bone model obtained in the bone model producing step; and a bone filler producing step (step 3) for producing a bone filler (4) which is filled in the bone deficit cite based on the figure-forming material filled in the bone deficit site of the bone model in the figure forming material filling step. And by a treatment of filling this bone filler, the bone deficit site is recovered and cured. In the figure, reference numeral 5 shows a recovered bone. Note that, in Fig. 1, conceptual diagrams which explain the conditions of bone deficit models corresponding to each steps are shown on the right side of each steps.

### 3-1 Bone Model Producing Step (Step 1)

The bone model producing step is a step for producing a bone model. Basically, the bone model is not specifically limited if it is used for producing a patient's bone model, and a well known method for producing a bone model can be used as appropriate. The examples of the method for producing a bone model include: a method for producing a bone model by pouring raw material into a mold which is produced based on, for example, an X-ray image of a bone; and a method for producing a bone model by the rapid prototype method. A method for producing a bone model by the rapid prototype method is preferred, because the bone model is produced based on digital data.

In recent years, rapid prototyping apparatus for easily forming three-dimensional structures (shown in, for example, JP-T 2001-524897, JP-T 2003-531220, JP-T 2004-538191, JP-T 2005-503939, U.S. Pat. No. 5204055 specification, U.S. Pat. No. 5340656 specification, U.S. Pat. No. 5387380 specification, U.S. Pat. No. 6007318 specification, U.S. Pat. No. 6375874 specification, U.S. Pat. No. 5902441 specification and U.S. Pat. No. 6416850 specification. These are incorporated herein for reference purposes) and rapid prototype process are increasingly used. Three dimensional objects, such as prototype parts of apparatus, are used to examine the performance thereof. The examples of rapid prototype method include the stereolithgraphy method, the powder sintering method, the powder binding method, and the solid ground curing technology (SGC), which forms thin layers based on a cross sectional shape data to form three-dimension shape by laminating the thin layers. In the stereolithgraphy method, the shape of a molding object is duplicated by sequentially irradiating laser beam to liquid resin material (light curing resin), which is hardened when a predetermined light is irradiated thereto, based on irradiation patterns stored in a computer. The bone model of the present specification is preferred to be formed with a resin such as light curing resin. Note that the light curing resin and a light source which hardens the light curing resin disclosed in JP-A 2004-49877 can be used as appropriate. In the powder sintering method, three-dimensional molding object as an aggregate of powder material is formed by repeating a step of laminating powder material and discharging binding material for combining the powder material based on drawing patterns stored in a computer. The bone model of the present specification is preferred to be formed with polymers such as a thermoplastic resin or thermosetting resin. A bone model may be obtained by sintering once formed powder binding solid object in high temperature. In this case, thermosetting resin is preferred to be used as raw material. Metal fine powder may be used as the above powder. So the bone model of the present specification may be one or a mixture of more than one of: titanium, iron, aluminum, copper, silver, gold, nickel, lead, tin, and the like. Also, since alloyed metal such as platinum and palladium is hardened with irradiation of laser beam, the shape of a molding object is duplicated by sequentially irradiating laser beam to thin layers formed from these metal powders based on irradiation patterns stored in a computer.

In the present invention, a method which is based on the RP method modified as needed for producing a bone model is preferred to be adopted. In particular, a bone filler producing method comprising: a cross-sectional shape obtaining step for obtaining information on the cross-sectional shape of each layer by dividing three-dimensional shape of a patient's bone into multiple layers; a first cross-sectional figure forming step comprising the steps of: reading out information on the cross-sectional shape of a first layer from the information on the cross-sectional shape obtained in the cross-sectional shape obtaining step; and forming the first cross-sectional figure duplicating the cross-sectional shape by using a figure-forming composition based on the read out information; a second cross-sectional figure forming step comprising the steps of: reading out information on the cross-sectional shape of a second layer from the information on the cross-sectional shape obtained in the cross-sectional shape obtaining step, the second layer being located on the upper layer of the first cross-sectional figure; and forming the second cross-sectional figure so as to be overlapped with the first cross-sectional figure, the second cross-sectional figure duplicating the cross-sectional shape from a figure-forming composition based on the read out information; and a three-dimensional figure obtaining step for obtaining the three-dimensional figure duplicating the shape of the object, wherein the three-dimensional figure obtaining step repeats, an upper layer cross-sectional figure forming step for forming an upper layer cross-sectional figure, in the same way as the second cross-sectional figure forming step, of: reading out information on the cross-sectional shape of the layer to be formed from the information on the cross-sectional shape obtained in the cross-sectional shape obtaining step; and forming the cross-sectional figure of the layer so as to be overlapped with the cross-sectional figure obtained in the former cross-sectional figure forming step, the cross-sectional figure of the layer duplicating the cross-sectional shape from a figure-forming composition based on the read out information, wherein at least one or more of the cross-sectional figure forming steps comprise: a figure-forming composition layer obtaining step for forming figure-forming composition layers by stratifying powders of the figure-forming composition; and a water adding step for moistening a predetermined part of the figure-forming composition layer by adding water to the figure-forming composition layer based on information on the cross-sectional shape of the layer, the layer formed in the figure-forming composition layer obtaining step.

A well known figure-forming composition can be used as appropriate. But a preferred example of the figure-forming composition is a figure-forming composition which is a mixture of calcium-based material and polyvinyl alcohol resin, wherein the polyvinyl alcohol resin is 2 to 8 weight parts when the total weight of the calcium-based material and the polyvinyl alcohol resin is 100 weight parts.

A preferred embodiment of the figure-forming composition is a figure-forming composition comprising a calcium-based material, a polyvinyl alcohol resin, and a hardening accelerator, wherein the hardening accelerator is one or more kinds of hardening accelerators selected from the group consisting of "dihydrate gypsum, alkali metal sulfate, alkaline earth metal sulfate, alkali metal chloride salt, alkaline earth metal chloride salt, inorganic acid ammonium salt, and alums", wherein the polyvinyl alcohol resin is 2 to 8 weight parts and the hardening accelerator is 0.1 to5 weight parts when the total weight of the calcium-based material and the polyvinyl alcohol resin is 100 weight parts. It is preferred that the figure-forming composition according to this embodiment exclusively comprise calcium-based material, polyvinyl alcohol resin, and hardening accelerator.

It is preferred that these figure-forming composition does not practically contain water except crystal water, and the one in powdered shaped is preferred. As far as figure-forming composition for building material is concerned, particle size of gypsum powder, which is raw material of the figure-forming composition, will not be a problem because gypsum powder is dissolved sufficiently by being mixed with water and the like. But, since the figure-forming composition of the present invention is not always intended to be in the form of slurry, the particle size of calcium-based material powder is preferred to be almost equalized. From this perspective, equal to or more than 50 weight parts of the molecule of the calcium-based material of the present invention should be located in the range of plus/minus 10 percent of the maximum distribution, according to a measurement of particle distribution based on JISR1619 (Testing method for size distribution of fine ceramic particles by liquid photosedimentation method). It is preferred that equal to more than 70 weight parts, more preferably equal to more than 85 weight parts, and further preferably equal to more than 95 weight parts thereof be located in the range of plus/minus 10 percent of the maximum distribution. This distribution can be achieved by repeatedly sorting out the ingredient powders.

A preferred example of calcium-based material of the figure-forming composition is gypsum. And examples of gypsum include α-type hemihydrate gypsum, β-type hemihydrate gypsum, or mixture of both. Among them, α-type hemihydrate gypsum is preferred. This is because, compared with β-type hemihydrate gypsum, α-type hemihydrate gypsum can achieve a kneaded state with little water, and hardening can be promoted. Hemihydrate gypsum having a small repose angle (repose angle is the maximum angle of inclination at which powders can form a stable slope) is preferred to be used, because the powders can be spread uniformly at the time of molding. From this perspective, the repose angle of hemihydrate gypsum (or figure-forming composition) is in the range of 30 to 45 degree, preferably 35 to 40 degree.

The polyvinyl alcohol resin of the present invention is not specifically restricted, and publicly known polyvinyl alcohol resin (polyvinyl alcohol (-[C(OH)HCH₂]ₙ-) or polyvinyl alcohol resin having a functional group as appropriate) can be used as needed. As the polyvinyl alcohol resin, saponified material (which is produced by saponifying lower alcohol solution of polyvinyl acetate with saponifying catalyst such as alkali or acid, in general) or derivative thererof can be used. Also, as polyvinyl alcohol resin, monomer which copolymerized with vinyl acetate, and saponified material which is a copolymer with vinyl acetate can be used. The examples of monomer which copolymerized with vinyl acetate include: olefine such as ethylene, propylene, isobutylene, α-octene, α-dodecen, and α-octadecene, unsaturated acids such as acrylic acid, methacrylic acid, crotonic acid, maleic acid, maleic anhydride, and itaconic acid or the salt thereof, or monoalkyl or dialkyl ester, nitriles such as acrylonitrile or meta acrylonitrile, amides such as acrylic amide, and methacrylamide, olefine sulfonic acid such as ethylene sulfonic acid, allyl sulfonic acid, and meta allyl sulfonic acid or the salt thereof , alkyl vinyl ethers, N-acrylic amide methyl trimethyl ammonium chloride, allyl trimethyl ammonium chloride, dimethyl diallyl ammonium chloride, dimethyl allyl vinyl ketone, N-vinyl pyrrolidone, vinyl chloride, vinylidene chloride, polyoxyalkylene (meta) allyl ether such as polyoxyethylene (meta) allyl ether and polyoxypropylene (meta) allyl ether, polyoxyalkylene (meta) acrylate such as polyoxyethylene (meta) acrylate and polyoxypropylene (meta) acrylate, polyoxyalkylene (meta) acrylic amide such as polyoxyethylene (meta) acrylic amide, and polyoxypropylene (meta) acrylic amide, polyoxyethylene (1- (meta) acrylic amide -1, 1- dimethylpropyl) ester, polyoxyethylene vinyl ether, polyoxypropylene vinyl ether, polyoxyethylene allylamine, polyoxypropylene arylamine, polyoxyethylene vinyl amine, polyoxypropylene vinyl amine. Preferably, saponified material of homopolymer of the vinyl acetate, saponified substance of copolymer of vinyl acetate and ethylene, unsaturated acid or the salt thereof, alkyl ester and olefine sulfonic acid or the salt thereof are used.

The figure-forming composition of the present invention, different from building materials and the like, is not needed to be placed in a mold nor kneaded. So, saponification degree and average degree of polymerization of polyvinyl alcohol resin is not specifically restricted. On the other hand, since mechanical strength of a three-dimensional figure is not improved with less than 70 mol percent of saponification degree, the saponification degree is preferred to be equal to or more than 70 mol percent, and more preferably it is in the range of 80 to 99.5 mol percent. If the average polymerization degree of polyvinyl alchol resin is below 2x10², the viscosity of slurry becomes too low. In contrast, if the average polymerization degree of polyvinyl alchol resin is over 3x10³, the viscosity of slurry becomes too high, which makes it difficult to be dissolved in water. So, the range of polymerization degree is for example 2x10² to 3x10³, and it may also be 5x10² to 2.5x10³. It may also be, for example, 3x10³ to 1x10⁴, because the figure-forming composition of the present invention is not needed to be placed in a mold nor kneaded. Also, if the polymerization degree is low, when water is added to the figure-forming composition and make it in the form of slurry, gypsum particles are settled out therein. But the figure-forming composition of the present invention is not need to be in the form of slurry. Since it is preferred that the polymerization degree be low and be easily dissolved in a little amount of water, the polymerization degree is, for example, 5x10 to 1.9x10², and it may also be 1x10² to 1.5x10². The polymerization degree or molecular weight can be controlled by adjusting reaction time or conditions as appropriate based on publickly known method.

Concerning polyvinyl alcohol resin, saponification degree of complete saponification type is, for example, in the range of 90 to 99.5 mol percent both inclusive. And more preferably the range is 98.5 to 99.5 mol percent both inclusive. As for viscosity thereof, 1x10 to 2x10 mPa·s is preferred. The viscosity is preferred to be measured based on JIS standard (e.g. JIS K 7367).

Note that polyvinyl alcohol resin may be polyvinyl alcohol resin itself, and it may be the resin of the polyvinyl alcohol derivative introducing a functional group as appropriate. Also, the functional group may be partially introduced thereto. And the polyvinyl alcohol resin may be a mixture of several kinds of polyvinyl alcohol resin. The examples of the functional group include an acetoacetyl group, a silyl group, a quaternary ammonium base, a carboxylic acid group, an inorganic base of carboxylic acid, a sulfonic group, an inorganic base of the sulfonic acid, a ketone group, a mercapto group, and an amino group. One or more than one kind of the above functional groups may be included. Among them, an acetoacetyl group or a silyl group is preferred, and the most preferred one include an acetoacetyl group as a functional group. Note that all the hydroxyl groups (-OH) may be substituted with functional groups, 5 to 95% of the hydroxyl groups may be substituted with functional groups, and 10 to 20%, 70 to 90%, or 30 to 70% of the hydroxyl groups may be substituted with functional groups. In particular, polyvinyl alcohol resin having an acetoacetyl group forms chelate with a metal ion which is contained in hardening accelerator, thereby achieving a prescribed hardness with little amount of water in a short period. These functional groups can be introduced to polyvinyl alcohol resin obtained as appropriate, based on a general method of organic synthesis. The kind or ratio of functional groups introduced can also be controlled based on a general method of organic synthesis.

The polyvinyl alcohol resin is mixed with the calcium-based material so that the polyvinyl alcohol resin is 2 to 8 weight parts per 100 weight parts of the total of the calcium-based material and the polyvinyl alcohol resin. As demonstrated in the example below, polyvinyl alcohol resin is preferred to be in the range of 3 to 7 weight parts. It may also be in the range of 3 to 6 weight parts, or 4 to 7 weight parts. It may further be in the range of 4 to 6 weight parts, or 4.5 to 5.5 weight parts. A suitable level of hardness can not be achieved with little amount of polyvinyl alcohol resin.

The figure-forming composition of the present invention may include the polyvinyl alcohol resin which is separate from calcium-based material, or it may be a mixture of calcium-based material and polyvinyl alcohol resin. In both cases, the figure-forming composition is preferred to be in a powdered state, and the size of the powders are preferred to be in the range as above described.

The hardening accelerator of the present invention is one or more than one kind of hardening accelerator selected from a group consisting of: dihydrate gypsum, alkali metal sulfate, alkaline earth metal sulfate, alkaline metal chloride salt, alkaline earth metal chloride salt, inorganic acid ammonium salt, and alums. The examples of alkali metal sulfate include sodium sulfate and potassium sulfate. The examples of alkali earth metal sulfate include magnesium sulfate, calcium sulfate and barium sulfate. The examples of alkali metal chloride salt include lithium chloride, sodium chloride and potassium chloride. The examples of alkaline earth metal chloride salt include magnesium chloride and calcium chloride. The example of inorganic acid ammonium salt includes ammonium hydrochloride. The examples of alums include potassium alum such as aluminum potassium sulfate 12 water: A1K (SO₄)₂·12H₂O, sodium alum such as A1Nₐ (SO₄)₂·12H₂O, ammonium alum such as NH₄Al (SO₄)₂·12H₂O. Among them, one or more than one kind selected from a group consisting of magnesium sulfate, sodium chloride, sodium sulfate, and calcium sulfate can preferably be used. And, a mixture of dihydrate gypsum; and one kind or more than on kind selected from a group consisting of magnesium sulfate, sodium chloride, sodium sulfate, and calcium sulfate can preferably be used. Also, a hardening accelerator having metal salt is preferred, because it forms a chelate structure with polyvinyl alcohol having a predetermined functional group, and improves the hardness of three-dimensional figures or three-dimensional structures.

When the hardening accelerator is added to the mixture of calcium-based material and polyvinyl alcohol resin, 0.1 to 5 weight parts of the hardening accelerator is preferred to be added to 100 weight parts of the total of calcium-based material and polyvinyl alcohol resin. The amount of dihydrate gypsum as hardening accelerator is, for example, 0.5 to 5 weight parts. On the other hand, the amount of hardening accelerator contained not having dihydrate gypsum is, for example, 0.1 to 5 weight parts, preferably 0.1 to 3 weight parts, further preferably 0.3 to 2 weight parts, and more preferably 0.4 to 1.5 weight parts, per 100 weight parts of the total amount of hemihydrate gypsum and polyvinyl alcohol resin.

The hardening accelerator is preferred to be mixed with figure-forming composition according to a publicly-known method in the field of figure-forming composition. The figure-forming composition of the present invention may include known compositions other than the ones above described as far as it retains the function of the figure-forming composition of the present invention.

In the method for forming a three-dimensional figure used for forming a bone model, basically, one of the above described figure-forming compositions which is in powdered state is used, when a three-dimensional figure is produced according to the rapid prototype process (the RP process). By using the above described figure-forming composition, even if a three-dimensional figure is formed by accumulating multiple layers to which a little amount of water (water, cross-linker solution, publicly known binder aqueous solution used for RP apparatus) was added, a three-dimensional figure having enough hardness to maintain a tentative form can be formed in a short period. It is also preferred that a unified three-dimensional figure be formed from adhesively joined layers each of which have certain level of hardness by being added with a small amount of water. The three-dimensional figure having the above characteristics can not be obtained by using the existing figure-forming composition as it is. But, by using the figure-forming composition of the present invention, a method for forming a three-dimensional figure according to this aspect can be obtained.

In particular, the method for forming a three-dimensional figure used for forming a bone model is a method for forming a three-dimensional figure duplicating the shape of an object, the method comprising: a cross-sectional shape obtaining step (step A1) for obtaining information on the cross-sectional shape of each layer by dividing three-dimensional shape of the object into multiple layers; a first cross-sectional figure forming step (step A2-1) comprising the steps of: reading out information on the cross-sectional shape of a first layer from the information on the cross-sectional shape obtained in the cross-sectional shape obtaining step; and forming the first cross-sectional figure duplicating the cross-sectional shape by using a figure-forming composition based on the read out information; a second cross-sectional figure forming step (step A2-2) comprising the steps of: reading out information on the cross-sectional shape of a second layer from the information on the cross-sectional shape obtained in the cross-sectional shape obtaining step, the second layer being located on the upper layer of the first cross-sectional figure; and forming the second cross-sectional figure so as to be overlapped with the first cross-sectional figure, the second cross-sectional figure duplicating the cross-sectional shape from a figure-forming composition based on the read out information; a three-dimensional figure obtaining step (step A2-n) for obtaining the three-dimensional figure duplicating the shape of the object, wherein the three-dimensional figure obtaining step repeats, an upper layer cross-sectional figure forming step for forming an upper layer cross-sectional figure, in the same way as the second cross-sectional figure forming step, of: reading out information on the cross-sectional shape of the layer to be formed from the information on the cross-sectional shape obtained in the cross-sectional shape obtaining step; and forming the cross-sectional figure of the layer so as to be overlapped with the cross-sectional figure obtained in the former cross-sectional figure forming step, the cross-sectional figure of the layer duplicating the cross-sectional shape from a figure-forming composition based on the read out information, wherein at least one or more of the cross-sectional figure forming steps comprise: a figure-forming composition layer obtaining step for forming figure-forming composition layers by stratifying one of the powders of the figure-forming composition above described; and a water adding step for moistening a predetermined part of the figure-forming composition layer by adding water to the figure-forming composition layer based on information on the cross-sectional shape of the layer, the layer formed in the figure-forming composition layer obtaining step. Hereinafter, each step is explained.

A cross-sectional shape obtaining step (step A1) is a step for obtaining information on the cross-sectional shape of each layer by dividing three-dimensional shape of the object into multiple layers based on information on the three-dimensional shape of the object. A preferred embodiment of the method for forming a three-dimensional figure used for forming a bone model is the above described method for forming three-dimensional figures, wherein the information on the cross-sectional shape of each layer comprises color identification information of each layer, and wherein water including coloring component is added based on the color identification information in the water adding step.

The method for forming three-dimensional figures used for forming a bone model can be easily performed by a publicly known apparatus used in, what is called, rapid prototype process, which is programmed to perform the steps of the method. In particular, the method is performed easily by using a computer which is programmed for rapid prototype process. This computer comprises an input/output part, a control part such as a CPU, a computing part, and a memory part. And it is connected with three-dimensional figure forming part for forming three-dimensional figures via an input/output part such as an interface. And the three-dimensional figure forming part comprises: a movable table for moving upward and downward to form a three-dimensional figure based on directions from the computer; a figure forming composition layer forming part for forming figure forming composition layer, by taking out figure forming composition powers from figure forming composition powder storing part in order to form figure forming composition layers on the movable table based on the orders from the computer; and a printing part for adding water or prescribed aqueous solution to the figure forming composition layer based on the orders from the computer.

In the cross-sectional shape obtaining step, it is preferred to obtain information concerning three-dimensional shape of an object, and then obtain images divided in cross-sectional shape composed of several layers of the three-dimensional shape. Also, the three-dimensional shape of implants and artificial bones can be obtained, for example, in the following way. In the first place, in order to obtain implants or artificial bones which fill defective sites, information on three-dimensional shape of the object may be obtained by computer simulating the shape of the bone of the object site so that the bones become in contrast. This is because a defective site generally has a counterpart whose shape is nearly in contrast with the defective site (for example, a right foot bone and a left hoot bone). Also, there are cases, such as manufacturing dental implants, that the shape of a diseased part itself is not suitable for duplicating. In this case, the shape of the object is drawn by 3DCG (three-dimensional computer graphics) based on the shapes of surrounding teeth and bones, and a computer obtains the information on the three-dimensional shape by inputting the 3DCG information, then information on each cross-sectional shape may be obtained by the computer based on the three-dimensional shape. In particular, when a signal from a pointing device is inputted in a CPU, the CPU reads out controlling program stored in the memory part such as CD-ROM or a hard disc based on the inputted signal. And the CPU scans X-ray figure stored in the memory part based on a direction from the controlling program, and a figure related to the three-dimensional shape is obtained by gathering a plurality of scanned two-dimensional figures. Note that since the X-ray photograph of bone or tooth part, and that of flesh part (or nervous part) are different in contrasting density, when the figure is obtained by scanning the X-ray photograph, an outline may be obtained from parts largely different in contrasting density. Also, patterning information of the bone part and the flesh part may be stored by obtaining the information, which is obtained by evaluating whether the contrasting density of the parts surrounded by the outline is in the range of predetermined value, or by comparing contrasting density of the part surrounded by the outline. Furthermore, when a figure concerning three-dimensional shape is obtained, the three-dimensional shape is, for example, sliced in the direction of Z-axis (the direction from the earth to the air), thereby obtaining cross sectional shape of each of a plurality of layers.

When contour lines are drawn on the surface of a bone model, contour lines corresponding to each height should be drawn by analyzing the height based on the shape of an obtained object (the shape of a bone model to be produced) by well-known methods. The rapid prototype method is to produce three-dimensional figures by laminating pluralities of layers. So, when each layer is produced, the top part and the bottom part of each layer may be colored. Also, for example, it is preferred to provide a colored layer every 2 to 100 layers. In this way, a bone model whereon contour lines are drawn every proper height can be obtained. Concretely, a predetermined part of each layer is colored with a coloring agent or an ink. It may also produce a colored layer by producing a bone model based on a program which is to form layers whereto a coloring agent and the like is added every predetermined layer.

When contour lines are drawn on the surface of a bone model, for example, a program in which, an obtained bone model is set so that (the front view of) the three dimensional data of the bone model is shown on the two-dimensional monitor, and grid are applied to the two dimensions data, then the grid are drawn on the surface thereof, is preferred to be used.

The thickness of the layers may be adjusted as appropriate according to input information from the pointing device and the like. It may also be controlled according to a preset value. If the thickness of the layer is too thick, an elaborate hardening body cannot be obtained, and there is a problem that the hardness for maintaining the shape cannot be achieved by adding water drops thereto by using devices such as printing mechanism. On the other hand, if the thickness of the layer is too thin, too many cross-sectional figures must be obtained, thereby causing a burden on the computer hardware resource, and too much time is required for forming a figure. From this perspective, the thickness of each layer is, for example, 1x10 µm to 5 mm. It may be 1x10 µm to 5 mm, or may be 1x10² µm to 1 mm. Note that the thickness of each layer is preferred to be uniform, but may not be uniform.

The first cross-sectional figure forming step (step A2-1) comprises the steps of: reading out information on the cross-sectional shape of a first layer from the information on the cross-sectional shape obtained in the cross-sectional shape obtaining step; and forming the first cross-sectional figure duplicating the cross-sectional shape by using a figure-forming composition based on the read out information.

The second cross-sectional figure forming step (step A2-2) comprises the steps of: reading out information on the cross-sectional shape of a second layer from the information on the cross-sectional shape obtained in the cross-sectional shape obtaining step, the second layer being located on the upper layer of the first cross-sectional figure; and forming the second cross-sectional figure so as to be overlapped with the first cross-sectional figure, the second cross-sectional figure duplicating the cross-sectional shape from a figure-forming composition based on the read out information.

Next, wherein the three-dimensional figure obtaining step repeats, an upper layer cross-sectional figure forming step for forming an upper layer cross-sectional figure, in the same way as the second cross-sectional figure forming step, reading out information on the cross-sectional shape of the layer to be formed from the information on the cross-sectional shape obtained in the cross-sectional shape obtaining step; and forming the cross-sectional figure of the layer so as to be overlapped with the cross-sectional figure obtained in the former cross-sectional figure forming step, the cross-sectional figure of the layer duplicating the cross-sectional shape from a figure-forming composition based on the read out information.

A method for forming a three-dimensional figure duplicating a shape of an object, wherein at least one or more of the cross-sectional figure forming steps comprise: a figure-forming composition layer obtaining step for forming figure-forming composition layers by stratifying powders of the figure-forming composition above described; and a water adding step for moistening a predetermined part of the figure-forming composition layer by adding water to the figure-forming composition layer based on information on the cross-sectional shape of the layer, the layer formed in the figure-forming composition layer obtaining step.

Hereinafter, examples of each cross-sectional figure forming step is explained. In each cross-sectional figure forming step, the CPU receive a direction from the controlling program, and read out information on the thickness of a figure-forming composition layer, then outputs the information from the input/output device. The three-dimensional figure forming part having received the information on the thickness shifts the movable table downward following an order from the computer. The downward shift distance corresponds to the thickness of the figure-forming composition layer. The information on the downward shift distance is also outputted from the computer. And the movable table moves based on the shift distance information. Note that if the thickness of each layer is the same, the memory part of the three-dimensional figure forming part stores this information, and may use the same information in forming each layer.

Next, the CPU receives the direction from the controlling program, and, for example, reads out information on the thickness of the figure-forming composition layer, computes the amount of figure-forming composition suitable for forming the figure-forming composition layer, then outputs the information of the amount from the input/output part. This amount may be fixed, and having transmitted to the three-dimensional figure forming part, it may be stored in the store part of the three-dimensional figure forming part, and the same information may be used for forming each layer. The three-dimensional figure forming part which have received information on this figure-forming composition layer, based on the direction from the computer, makes the figure-forming composition layer forming part take out figure-forming composition powder from the figure-forming composition powder storing part, then releases the powder on the table. The three-dimensional figure forming part may control the figure-forming composition layer forming part to uniform the figure-forming composition layer by moving a squeezee or a spatula. In this way, the figure-forming composition layer is formed on the movable table (if already a layer is formed, another layer will be formed on a figure-forming composition layer formerly formed).

Next, having received a direction from the controlling program, the CPU reads out information on the cross-sectional shape of each layer or information on pattering, and outputs the information from the input/output part. The three-dimensional figure forming part, based on a direction from the computer, activates a printing part, and adds water or predetermined aqueous solution (water, cross-linker solution, binder aqueous solution for rapid prototyping) to the figure-forming composition layer. This mechanism can be easily achieved by using a controlling mechanism of a well known printer. Note that the conditions such as the composition, the density, and the amount of water or aqueous solution to be added can be adjusted as appropriate. For example, information on these conditions is inputted from the pointing device, and the inputted information is stored in the store part of the computer. Based on the information on these conditions, the CPU reads out necessary information and makes the computing part to perform computing, and controls the operation of the printing part. The printing part uses ordinary printing techniques except adding water instead of ink. The liquid binder material added to the figure-forming composition layer may be organic or inorganic. Typical organic binder material used is a ceramic precursor such as polymer resin or polycarbosilazane. Inorganic binder is used when a binder is mixed with the final material, in which silica is generally used.

Ordinarily, in the step of forming each layer, the amount of water which is more than the amount necessary for accelerating hydration reaction is repeatedly added and dried. But, in the method for forming three-dimensional figure of the present invention (the method for forming hardening material of the present invention), the hydration reaction of gypsum is not need to be completed in the above step. So, in each cross sectional figure-forming step, for example, when the amount water necessary for hydrating the figure-forming composition completely is assumed to be 100 weight parts, the amount of water to be added may be, for example, 1 to 50 weight parts, 1 to 20 weight parts, 2 to 10 weight parts, or 3 to 5 weight parts. This little amount of water is not sufficient for completing the hydration reaction of gypsum. However, in the present invention, with this little amount of water, layers with hardness which is enough for maintaining least hardness can be obtained rapidly. Also, since the amount of water is little, the water can be prevented from spreading to unintended part, thereby obtaining layers having desired cross-sectional structures. In particular, in case of obtaining a cross-sectional structure which has more than two kinds of patterning, it is necessary for preventing two kinds of water or aqueous solution from being mixed. With little amount of water to be added, these two kinds of water or aqueous solution can be prevented from being mixed.

Having repeatedly performed the cross-sectional figure forming step, it is preferred to dry the resultant layered product until it has a certain level of hardness. The drying may be performed in low humidity high temperature atmosphere (for example humidity 0 to 10%, temperature 50 to 2x10²°C), but may be performed at ordinary temperatures and pressures. The drying time at an ordinary temperature and pressure is preferred to be adjusted as appropriate, according to the size, the moisture percentage, and the thickness of each layer of the resultant three-dimensional figure. The examples of the drying time include 1 minute to 1 hour, 5 minutes to 3x10 minutes, and 5 minutes to 2x10 minutes. Namely, in using the rapid prototype process in the present invention, figure-forming composition containing a large amount of polyvinyl alcohol resin is used, so a figure-forming composition having relatively high level of hardness could be obtained. And in this step, the figure-forming composition need not contain enough water, so drying time can be remarkably shortened. Then, after drying the layered product, a three-dimensional figure duplicating the shape of an object can be obtained. Note that, the drying is preferred to be performed in a deaeration step, for example deaeration by decompression, because in the deaeration step, the drying can be performed quite rapidly.

It is highly likely that the three-dimensional figure obtained in the above way contains gypsum whose hydration reaction does not proceeded. So, the hardness thereof is assumed to be low compared to that of three-dimensional figure whose hydration reaction is preceded. However, by patterning layers with a little amount of water, water can be prevented from spreading to unintended parts, thereby preventing the unintended parts from being hardened. So, this method for forming three-dimensional figure is useful for forming three-dimensional figure having a sophisticated shape in a short period. On the other hand, the three-dimensional figure obtained in the above way has a sophisticated shape, but it is assumed that the hardness thereof is low because the hydration reaction is not sufficiently preceded. In order to obtain enough hardness, it is preferred that hydration reaction be proceeded according to the method for forming hardening object described later.

As above described, the method for producing a bone model is the method for obtaining hardening body having enough hardness basically by the following procedures. The resultant three-dimensional figure obtained in the above each step is soaked into water or aqueous solution, thereby accelerating hydration of gypsum. And then the resultant figure is dried.

Namely, the method for producing a bone model relates to the method comprising: a gypsum powder removing step (step B1) for removing unconsolidated figure-forming composition powder from the three-dimensional figure basically obtained by one of the method for forming a three-dimensional figure above described; a water adding step (step B2) for adding water to the three-dimensional figure whose unconsolidated powders were removed in the gypsum powder removing step; and a drying step (step B3) for drying the three-dimensional figure to which water was added in the water adding step. Hereinafter, each step is explained.

Gypsum powder removing step (step B1) is a step for removing powders of unhardened figure-forming composition from the three-dimensional figure. In this step, for example, unhardened gypsum powders are blown off by an airbrush. The amount of airflow, the shape of the airbrush, and the like may be adjusted as appropriate, and a well known airbrush can be used. The time required for the gypsum powder removing step is also adjusted as appropriate. The specific example is 5 minutes to 1 hour, and 10 minutes to 30 minutes is preferred.

Water adding step (step B2) is a step for adding water to the three-dimensional figure whose powders are removed in the gypsum powder removing step. In this water adding step, enough water for accelerating hydration reaction of gypsum is preferred to be added to a three-dimensional figure. In this water adding step, three-dimensional figure is soaked in water or predetermined aqueous solution. In this process, since unnecessary powders are removed in the former gypsum powder removing step, figure-forming composition powders unnecessary for forming the shape of the three-dimensional figure can be prevented from sticking to the three-dimensional figure.

A preferred embodiment of the method for forming three-dimensional structures according to the third aspect of the present invention includes the water adding step (step B2) which comprises: an atomizing step (step B2-1) for attaching water on the surface of the three-dimensional figure by misting water or by exposing the three-dimensional figure to high humidity atmosphere, the three-dimensional figure being removed unconsolidated powders therefrom in the gypsum powder removing step; and a soaking step (step B2-2) for soaking the three-dimensional figure in water after the atomizing step.

There is a problem that the shape of three-dimensional figure is deformed if the resultant three-dimensional figure is soaked in water suddenly. Considering this problem, the following procedures are taken in this embodiment of the method for forming three-dimensional structure. Firstly, water is added on the surface (preferably on all over the surface) of the resultant three-dimensional figure, thereby accelerating the hardening reaction, at least, on the surface thereof through hydration reaction of gypsum (preferably followed by drying the figure) and preventing the figure from being deformed. Then, the hardening reaction is further accelerated by soaking the figure in water. In the atomizing step, for example, water or predetermined aqueous solution (preferably water, cross-linker solution, or binder aqueous solution) is sprayed on the surface of the three-dimensional figure by using a known spray. Or water is added on the surface of the three-dimensional figure by placing the three-dimensional figure in high humidity atmosphere. Then, having sprayed water, the figure is dried, and then soaked in water. The figure may be dried in low humidity high temperature atmosphere (for example humidity 0 to 10%, temperature 50 to 2x10²°C), but may be dried at an ordinary temperature and pressure. The drying time at an ordinary temperature and pressure is preferred to be adjusted according to the size, the moisture percentage, and the thickness of each layer of the resultant three-dimensional figure, as appropriate. The examples of the drying time include 1x10 minutes to 2 hours, 15 minutes to 1 hour, and 2x10 minutes to 4x10 minutes. In the soaking step, the three-dimensional figure is soaked in sufficient water or aqueous solution. The soaking time may be adjusted as appropriate according to the size of the three-dimensional figure. The examples of the soaking time include 1x10 minutes to 2 hours, 15 minutes to 1 hour, and 2x10 minutes to 4x10 minutes.

A preferred embodiment of the method for producing a bone model is the above described method for producing a bone model comprising the water adding step (step B2) which comprises: (1) an atomizing step for attaching water on the surface of the three-dimensional figure by misting water or by exposing the three-dimensional figure to high humidity atmosphere, the three-dimensional figure being removed unconsolidated powders therefrom in the gypsum powder removing step, and a soaking step for soaking the three-dimensional figure in cross-linker solution after the atomizing step; (2) an atomizing step for attaching cross-linker solution on the surface of the three-dimensional figure by misting cross-linker solution or by exposing the three-dimensional figure to high humidity atmosphere of cross-linker solution, the three-dimensional figure being removed unconsolidated powders therefrom in the gypsum powder removing step, and a soaking step for soaking the three-dimensional figure in cross-linker solution after the atomizing step; or (3) an atomizing step for attaching water on the surface of the three-dimensional figure by misting water or by exposing the three-dimensional figure to high humidity atmosphere, the three-dimensional figure being removed unconsolidated powders therefrom in the gypsum powder removing step, and a soaking step for soaking the three-dimensional figure in water and then in cross-linker solution after the atomizing step. In particular, step (1) or (3) is preferred to be used for figure-forming composition containing acetoacetyl group modified polyvinyl alcohol resin. This is because, in view of hardness or uniformity of the three-dimensional structure, it is preferred to promote bridging reaction with a cross-linker, after having developed chelate structures with water.

In this way, by adding a cross-linker such as cross-linker solution, cross-linking reaction proceeds in the three-dimensional figure, and a three-dimensional structure having enough hardness can be obtained. The atomizing step and the soaking step are performed in the same way as above explained. The density of cross-linker solution is adjusted as appropriate according to the kind of polyvinyl alcohol resin used and the hardness of the hardening body to be obtained. The concentration of cross-linker solution is specifically 1x10⁻² to 2x10 volume percent, preferably 1x10⁻¹ to 1.5x10 volume percent. As cross-linker solution, in place of or together with amine cross-linker solution such as ethylenediamine or diethanolamine, the following materials can be used as appropriate: aldehyde compound such as formaldehyde or glyoxal; methylol compound such as melamine-formaldehyde condensate or urea-formaldehyde condensate; boron-containing compound such as boracic acid or borax; isocyanate compound such as 2,4-tolylene diisocyanate, 2,6-tolylene diisocyanate, m-phenylene diisocyanate, p-phenylene diisocyanate or 4,4-diphenylmethane diisocyanate; or silane coupling agent. Among them, as a cross-linker, amine cross-linker solution such as ethylenediamine or diethanolamine is preferred. In particular, as demonstrated in the example described later, one or both of ethylenediamine and diethanolamine is more preferred.

The drying step (step B3) is a step for drying the three-dimensional figure which is added water in the water adding step. The drying may be performed in low humidity high temperature atmosphere (for example, humidity 0 to 10%, temperature 50 to 2x10²°C), but may be performed at an ordinary temperature and pressure. The drying time at an ordinary temperature and pressure is preferred to be adjusted according to the size, the moisture percentage, and the thickness of each layer of the resultant three-dimensional figure, as appropriate. The examples of the drying time include 1 hour to 4 days, 4 hours to 3 days, and 6 hours to 2 days.

In the present invention, however, the drying time is preferred to be from 1 hour to 4 hours, because the bone model is preferred to be produced relatively quickly.

### 3-2. Figure-forming Material Filling Step (Step 2)

The figure-forming material filling step is a step for filling figure-forming material in a bone deficit site of the bone model obtained in the bone model producing step. A figure of a bone filler can be obtained by filling figure-forming material in the bone deficit site.

Figure forming material is not specifically limited if a figure of a bone filler is obtained from the figure forming material. And a well known figure-forming material can be can be used as appropriate. But the figure forming material is preferably distinguished from a bone model. Specifically, figure forming material which differs from the bone model in one of the X-ray permeability, the infrared ray permeability, or the ultraviolet ray permeability is preferred. For example, the X-ray permeability, the infrared rays permeability, or the ultraviolet rays permeability of the figure of a bone filler whereon figure forming material is combined are equal to or less than 90% or equal to or more than 110% of those of a bone model. If the figure-forming material has the above X-ray permeability, infrared ray permeability, or ultraviolet ray permeability, a figure of a bone filler whereon figure forming material is combined can be easily distinguished from a bone model. The figure-forming material, for example, is made from raw material which is the same as that of a bone model including coloring agent, metallic powder, or metallic oxide powder

The figure-forming material, for example, contains wax ingredient (e. g., dental wax) as the main component. The examples of the wax ingredient, other than paraffin wax, include one or a mixture of:; beeswax; microcrystalline wax; dammar; rosin; candelilla wax ; carnauba wax; or montan wax. Among them, the one which mainly contains paraffin wax is preferred. The examples of the wax component of the figure-forming material containing paraffin wax as the main component include: wax component having flexibility and adhesiveness by adding beewax or microcrystalline wax thereto; wax component having improved strength and hardness as well as having adhesiveness by adding dammar or rosin thereto; and wax component having glazing surface by adding carnauba wax which has high-melting point thereto. Another example is a synthetic resin which is a mixture of hydrocarbon resin and ethylene-vinyl copolymer resin. In particular, ethylene-vinyl copolymer resin is mixed in paraffin wax so that the ethylene-vinyl copolymer resin is preferably 1 to 5 weight parts when the total weight is 100 weight parts. More specific examples of dental wax include paraffin wax, rolling wax, proline wax, corben wax, pro-utility wax, bite rim stick or carving wax. Number average molecular weight (Mn) measured by a gel permeation chromatography (GPC) is from 400 to 5,000, preferably from 800 to 5,000, more preferably from 1,000 to 3,000, further preferably from 1,500 to 2,500. The ratio between the weight-average molecular weight measured by a GPC and the number average molecular weight (Mw/Mn) of the wax is equal to or less than 4.0, preferably equal to or less than 3.5, more preferably equal to or less than 3.0. Note that weight average molecular weight (Mw) and the number average molecular weight (Mn) are polystyrene equivalent measured by gel permeation chromatography (GPC). The GPC measuring is performed under the following condition: temperature 140°C; solvent ortho-dichlorobenzene.

As for wax, other than generally used red dental wax, white wax, yellow wax, black wax, or the like can be used as appropriate. White wax is produced by mixing the titanium oxide, which is a white pigment, in wax such as the paraffin wax. Yellow wax, for example, is produced by mixing titan yellow, which is a kind of the titanium oxide (yellow pigment), in wax such as paraffin wax. Black wax, for example, is produced by mixing aniline black, which is a black pigment, in wax such as paraffin wax.

The figure-forming material is preferred to include inorganic powder, organic powder, surfactant metal salt powder, pigment, coloring agent, metallic powder, metallic oxide powder, or the like, so that a figure of a bone filler whereon figure-forming material is combined can be distinguished from a bone model in one of the X-ray permeability, the infrared ray permeability, or the ultraviolet ray permeability.

Specific examples of inorganic powder include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, lithia mica, silica acid, silicic anhydride, aluminium silicate, magnesium silicate, magnesium aluminium silicate, calcium silicate, barium silicate, strontium silicate, tungstic acid metal salt, hydroxyapatitte, vermiculite, higilite, bentonite, montmorillonite, hectorite, zeolite, ceramics powder, calcium secondary phosphate, alumina, aluminum hydroxide, boron nitride, and silica.

Specific examples of inorganic powder include polyamide powder, polyester powder, polyethylene powder, polypropylene powder, polystyrene powder, polyurethane, benzoguanamine powder, polymethyl benzoguanamine powder, tetrafluoroethylene powder, polymethyl methacrylate powder, cellulose, silk powder, nylon powder, 12 nylon, 6 nylon, crosslinkable silicone impalpable powder having a cross-linked structure of dimethyl silicone, impalpable powder of polymethyl silsesquioxane, styrene • acrylic acid copolymer, divinylbenzene • styrene copolymer, vinyl resin, urea resin, phenol resin, fluororesin, silicone resin, acrylic resin, melamine resin, epoxy resin, polycarbonate resin, mycrocrystallite fiber powder, starch powder, lauroyl lysine. Further, organic powder most of which is composed of - [Si-O-]ₙ- frame can be used. In this case, the molecule may have a -Si (CH₂CH₂)ₘ-Si- coupling therein.

Specific examples of surfactant metal salt powder (metal soap) include zinc stearate, aluminum stearate, calcium stearate, magnesium stearate, zinc myristate, magnesium myristate, zinc cetyl phosphate, calcium cetyl phosphate, Sodium zinc cetyl phosphate.

Specific examples of colored pigment include: inorganic red pigment such as iron oxide, iron hydroxide, and iron titanate; inorganic brown-based pigment such as the y - iron oxide; inorganic yellow-based pigment such as yellow iron oxide, and yellow ocher; inorganic black pigment such as black iron oxide, and carbon black; inorganic purple pigment such as manganese violet, and cobalt violet; inorganic green pigment such as chromium hydroxide, chromium oxide, cobalt oxide, and cobalt titanate; inorganic blue-based pigment such as iron blue, and ultramarine blue; laked tar-based pigment; laked natural pigment; and synthetic resin powder which combined powders thereof.

Specific examples of pearl pigment include titanium oxide coating mica, bismuth oxychloride, titanium oxide coating bismuth oxychloride, titanium oxide coating talc, fish scale guanine, titanium oxide coating coloration mica.

Specific examples of metallic powder pigment include aluminum powder, cupper powder, and stainless powder.

Specific examples of natural pigment is powder of carminic acid, laccaic acid, carthamin, brazilin, and crocin. These powders can be combined or can be processed with general oil, silicone oil, a fluorine compound, or surfactant as long as it maintains the effect of the present invention. Organohydrogen polysiloxane having reactiveness, organopolysiloxane having a hydrolysable alkoxysilane group, and acryl silicon-based copolymer having a hydrolsable silyl group can also be used. One or more than one kind of them can be mixed as needed. A specific example of the pigment is the above titanium oxide. Metallic powder or the powder of metallic oxide is not specifically limited, and a well-known metallic powder or the powder of metallic oxide can be used as appropriate. The preferred example of the metallic powder or the powder of metallic oxide is titanium oxide, more preferably titanium oxide of anatase type or rutile type. The particle size of the metallic powder or the powder of metallic oxide (analyzed based on JISR1619) is from 1x10²nm to 5x10³nm, preferably form 5x10²nm to 3x10³nm. As demonstrated in the example below, figure-forming material, for example, contains wax of more than 90 weight parts (preferably more than 95 weight parts) of the total amount, and contains the metallic powder or the powder of metallic oxide of 2 to 5 weight parts of the total amount, more preferably contains the metallic powder or the powder of metallic oxide of 3 to 4 weight parts of the total amount.

Since a bone model is formed based on the bone of a patient by the PR method and the like, surgeons and the like can fill figure-forming material into the bone deficit site of the bone model. In this way, since a practitioner oneself forms a figure of bone filler on the precisely formed bone model, a bone filler which is usable for a practitioner can be obtained. In particular, since the figure-forming material, for example, contains paraffin wax and the like as the main component, it easily becomes flexible in hot water. So, the figure-forming material having the flexibility is preferred to be filled in a bone deficit site. Also, when the figure-forming material is filled in a bone deficit site of a bone model, bone filler may be automatically filled in the deficit site of the bone model.

The figure-forming material filled in a deficit site of a bone model, for example, contains paraffin wax as the main component. So, it will be hardened to be a figure of a bone filler by leaving it as it is. This figure of a bone filler is used for treatment of a patient. And it precisely fits in the shape of a bone deficit site.

### 3-3. Bone Filler Producing Step (Step 3)

The bone filler producing step is a step for producing a bone filler which is filled in the bone deficit site based on the figure-forming material (or a figure of a bone filler) filled in the bone deficit site of the bone model in the figure-forming material filling step. Figure-forming material is filled in the bone deficit site of the bone model in the figure-forming material filling step, and the figure of the bone filler whereon figure-forming material is combined is distinguished from the bone model in some way. So, a bone filler is preferred to be produced by using this figure of bone filler. Also, a bone filler may be produced by the powder injection molding method or its modified method, by using a mold which is produced based on the shape of the obtained figure of a bone filler.

In particular, a bone filler is produced in the following way. Since a figure of a bone filler and a bone model has different X-ray permeability or reflectivity, each bone model containing a figure of a bone filler is photographed by an X-ray CT and the like. The obtained X-ray permeability or reflectivity is sent to a control equipment such as a personal computer. The control equipment grasps the shape of the figure of the bone filler based on the difference of an X-ray permeability or reflectivity, sends the information of the shape of the bone filler to a production unit of a bone filler, and outputs a direction to the production unit for producing a bone filler based on the shape of the bone filler. The production unit is, for example, a production unit based on the PR method, and a bone filler is produced by the PR method or the above explained method. Note that when a bone filler is produced by the PR method, compound powers (such as calcium phosphate-based material) which is explained below is preferred to be used as raw material powders as appropriate.

Hereinafter, a method for producing a bone filling material based on the powder injection molding method is explained. The method for producing a bone filling material of the present invention basically comprises the steps of: (a) kneading ingredient comprising calcium-based material and material comprising binder; (b) molding a molded body having a predetermined shape from a kneaded material obtained in step (a) with an injection molding machine having a mold; (c) removing the binder contained in the molded body (i.e., degreasing) to obtain a degreased body, the molded body being obtained in step (b); and (d) heating and sintering the degreased body to obtain a sintered body, the degreased body being obtained in step (c). The method may include publicly known steps such as an after treatment step for a molded body.

Since each of the bone filling material obtained by the metod for producing bone filling material has uniformity in size, appropriate dosage of pharmaceutical agent can be administered, even when the pharmaceutical agent is incorporated in the bone filling material. Furthermore, the bone filling materials, when administered, have appropriate porosity while maintaining the strength of each bone filling material, because the bone filling material has uniform density and its size can be controllable. Each steps of the method for producing a bone filling material is explained in the following.

In the kneading step, ingredient comprising calcium-based material and material comprising binder are kneaded. In this step, it is preferred to use powdered ingredient. In this step, powdered ingredient and sub materials such as binders are mixed so that they are made to be suitable for injection molding.

Calcium-based materials, for example, are used as powdered ingredients. Examples of the calcium-based materials include calcium phosphate-based materials, calcium carbonate-based materials, calcium lactate, and calcium gluconate. Among them, calcium phosphate-based material or calcium carbonate-based material is preferred. Specific examples of calcium phosphate-based materials as powdered ingredients include one or more than one kind of hydroxyl apatite, carbonic acid apatite, fluorapatite, chlorapatite, β-TCP, α-TCP, calcium metaphosphate, tetra-calcium phosphate, calcium hydrogen phosphate, calcium dihydrogen phosphate, calcium pyrophosphate, octacalcium phosphate, the salts thereof, and the solvates and dihydrates thereof. Among them, β-TCP or hydroxyl apatite is preferred. Specific examples of calcium carbonate-based materials include calcium carbonate and calcium hydrogen carbonate. Among them, calcium carbonate is preferred. Note that, the powdered ingredients are not specifically limited to these materials, and well-known materials used as ingredients of the bone filling materials can be used as appropriate.

When the size of the ingredient powder is too small, many binders are required for forming a mold, and the physical properties of the resultant bone filling material is deteriorated. On the other hand, when the size of the ingredient powder is too large, there are risks that the ingredient powders get into gaps between a screw and a cylinder of a molding machine, or sintering of ingredient powder does not proceed. In the present invention, powder injection molding is basically performed, but the ingredient powder used in the powder injection molding is not always metal powder. As a result of an experiment, the grain size of the ingredient powder is, for example, from 0.01 µm to 100 µm (both inclusive, same below), and is preferably from 0.1 µm to 20 µm. In a general powder metallurgy, for example, powders with the size of 100µm are used. For example, in Japanese Patent Laid-Open No. 2004-97259, hydroxyl apatite having a grain size of less than 150 µm is used (paragraph [0025]). But in the present invention, it is preferred that ingredient powder having relatively small grain size be used to improve fluidity of kneaded material (mixture of ingredient powders and binders) and improve the density of a sintered body. On the other hand, although the bone filling material of the present invention requires certain strength, it is assumed to be eroded by osteoclasts. From this perspective, the grain size may be from 0.1 µm to 50 µm, and may preferably be from 0.5 µm to 10 µm.

In the kneading step, materials such as binder, other than the ingredient, are mixed with the ingredient. The examples of the binder include (meta) acrylic-based resin, wax lubricant, (preferably, thermoplastic resin other than (meta) acrylic-based resin) and material having lubricant. The example of methacrylic-based resin or acrylic-based resin is methacrylate resin or acrylate resin, and it specifically includes a polymer of n-butyl methacrylate or methyl methacrylate, or a copolymer of n-butyl methacrylate and methyl methacrylate. The molecular weight of methacrylic-based resin or the acrylic-based resin is not specifically limited, but it is preferred to be adjusted as appropriate so that the physical properties of the resultant bone filling material are not lost, and the weight average molecular weight is, for example, 1x10³ to 1x10⁵. The content of methacrylic-based resin or acrylic-based resin in the binders is not specifically limited, but it is preferred to be adjusted as appropriate so that the physical properties of the resultant bone filling material are not lost, and the content is, for example, 1% by weight to 50% by weight.

The example of the wax lubricant is wax having a melting point of 40°C to 100°C, and the melting point is preferably 40°C to 70°C. As the wax having the above melting point, for example, well known paraffin wax can be used as appropriate. A molded body can be easily removed from a mold at the time of injection molding by using wax having the above melting point. It is more preferred to use wax having a melting point of 60°C to 65°C, because a molded body can be removed from a mold without cooling the mold too much.

The examples of wax lubricant include one or more than one kind of: hydrocarbon oil such as liquid paraffin, squalene, and squalane; higher fatty acid such as oleic acid, tall oil, and isostearic acid; higher alcohol such as lauryl alcohol, oleyl alcohol, isostearyl alcohol and the octyldodecanol; silicone oil such as methyl polysiloxane, methyl phenyl polysiloxane, methyl hydrogen polysiloxane, and decamethyl polysiloxane; ester such as isopropyl myristate, isopropyl palmitate, hexyl laurate, oleyl oleate, decyl oleate, octyl dodecyl myristate, hexyl decyl dimethyl octanoate, diethyl phthalate, and dibutyl phthalate; animal and plant oil such as avocado oil, camellia oil, turtle oil, macademia nut oil, corn oil, sesame oil, persic oil, wheat germ oil, sasanqua oil, castor oil, linseed oil, safflower oil, cotton oil, perilla oil, Chinese bean oil, peanut oil, tea seed oil, kaya oil, rice bran oil, jojoba oil, apricot kernel oil, olive oil, carrot oil, grape seed oil, rape seed oil, camellia oil, jojoba oil, egg yolk oil, lanolin oil, and mink oil; and glycerine such as glycerine, diglycerol, triglycerine, glycerine trioctanoate, and glycerine triisopalmitate. The melting point of wax lubricant can be controlled by adjusting molecular weight and composition ratio of these raw materials as appropriate.

The molecular weight of wax lubricant is not specifically limited, but it is preferred to be adjusted as appropriate so that the physical properties of the resultant bone filling material are not lost, and the weight average molecular weight is, for example, 1x10² to 1x10⁶. The content of wax lubricant in the binders is not specifically limited, but it is preferred to be adjusted as appropriate so that the physical properties of the resultant bone filling material are not lost, and the content is, for example, 1% by weight to 50% by weight.

The examples of a thermoplastic resin include one or more than one kind of: polyacetal resin, (meta) acryl resin, polyolefin resin, ethylene - vinyl acetate copolymer, and polyvinyl butyral. However, in the present invention, resins other than (meta) acrylate resin and (meta) acrylic - based resin are preferred as thermoplastic resins. Among them, ethylene - vinyl acetate copolymer is preferred.

The molecular weight of a thermoplastic resin is not specifically limited, but it is preferred to be adjusted as appropriate so that the physical properties of the resultant bone filling material are not lost, and the weight average molecular weight is, for example, 1x10³ to 1x10⁵. The content of a thermoplastic resin in the binders is not specifically limited, but it is preferred to be adjusted as appropriate so that the physical properties of the resultant bone filling material are not lost, and the content is, for example, 1% by weight to 50% by weight.

The examples of lubricant (other than wax lubricant) include one or more than one kind of: stearic acid, a salt of stearic acid, a hydrate of stearic acid, a hydrate of a salt of stearic acid, and C₁-C₅ alkyl stearic acid (C₁-C₅ alkyl represents an alkyl group having 1 to 5 carbon atoms, same below); or one of these materials and polyethylene glycol or one of these materials and polyglycerol. The content of lubricant in the binders is not specifically limited, but it is preferred to be adjusted as appropriate so that the physical properties of the resultant bone filling material are not lost, and the content is, for example, 0.5% by weight to 15% by weight. The molded bodies can be removed from a mold easily by using the above lubricant. The lubricant may also act as a dispersing agent.

The phthalic acid ester group is another compound comprising the binders. It is reported that the phthalic acid ester group is harmful to the human body. But in a preferred embodiment, the binders are thermally decomposed almost completely. So chemical compounds having poor biocompatibility, such as the phthalic acid ester group, can be contained in binders. The example of the phthalic acid ester group is C₁-C₅ alkyl phthalate such as dibutyl phthalate. A bone filling material having more preferred physical properties could be obtained by consciously using the phthalic acid ester group.

The molecular weight of the phthalic acid ester group is not specifically limited, but it is preferred to be adjusted as appropriate so that the physical properties of the resultant bone filling material are not lost, and the weight average molecular weight is, for example, 1x10⁴ to 1x10⁷. The phthalic acid ester group with poor volatility is also preferred. The content of the phthalic acid ester group in the binders is not specifically limited, but it is preferred to be adjusted as appropriate so that the physical properties of the resultant bone filling material are not lost, and the content is, for example, 0% by weight to 20% by weight, and is preferably 0.5% by weight to 15% by weight.

Binders are removed by being thermally decomposed in the binder removing step described below. The parts where binders existed basically become voids. So the porosity and the intensity of a resultant bone filling material can be adjusted by controlling the quantity of binders added to ingredient. But in general, the amount of binders required is the amount that is enough to fill spaces between particles of ingredients. This is because if the amount of binders is not enough, appropriate fluidity can not be obtained, which in turn causes injection molding defects such as short mold and weld, and also causes variations in the shape or the density of the resultant molded bodies. Binder Loadings are, for example, between 10 percent by weight to 100 percent by weight, based on 100 percent by weight of ingredients, or may be 20 percent by weight to 50 percent by weight. The blending ratio of binders to ingredients is 25 to 70 volume percent, preferably 30 to 55 volume percent, and more preferably 35 to 45 volume percent.

A preferred embodiment is a method for producing a bone filling material comprising "ingredient comprising calcium-based material, and material comprising binder", and glass component. As the glass component, the following materials can be used as appropriate: silica glass which is composed mostly of silicon dioxide; borosilicate glass containing 5 to 20 % by weight of B₂O₃; lead glass containing 5 to 40 % by weight of lead; potassium glass containing 5 to 30 % by weight of potassium; Fluoroaluminosilicate glass including sodium fluoride, aluminum fluoride, and the strontium fluoride; or a mixture of one of these glasses and one kind or a mixture of more than one kind of boric acid, lanthanum oxide, gadolinium oxide, niobium oxide, zirconium oxide, and barium. The sinterability of a sintered body is lowered by consciously adding glass components. As a result, minute cracks and porosities are formed on the surface or inside the sintered body, thereby producing a bon filling material which is suitable for culturing cells. As glass component, the following materials or an appropriate mixture thereof may be used: titanium, titanium alloy, cobalt - chromium alloy, stainless steel, alumina, zirconia. Calcium phosphate-based crystals such as apatite (Ca₁₀ (PO₄) ₆ O), or calcium phosphate-based crystals such as CaO-SiO₂-MgO-P₂O₅ based crystallized glass may also be used.

It is preferred that the glass component loadings are adjusted as appropriate based on the physical properties required for the bone filling material, but the loadings are, for example, between 1 percent by weight to 20 percent by weight, based on 100 percent by weight of ingredients, or may be 2 percent by weight to 10 percent by weight. The blending ratio of glass components to ingredients is 1 to 20 volume percent, preferably 2 to 10 volume percent, more preferably 3 to 10 volume percent.

A preferred embodiment is a method for producing a bone filling material comprising "ingredient comprising calcium-based material, and material comprising binder", and salt or sugar (preferably salt). The sinterability of a sintered body is lowered by consciously adding salt or sugar. As a result, minute cracks and porosities are formed on the surface or inside the sintered body, thereby producing a bone filling material which is suitable for cell culture. And, by immersing the obtained bone filling material in water, salt or sugar can be removed, thereby producing a bon filling material which is suitable for cell culture. Well-known salts or sugars can be used as appropriate. Salts which can be dissolved in water but is not thermally decomposed at temperature that thermally decomposes binders, particularly inorganic salt, is preferred. Specific examples of the salts include sodium chloride, potassium chloride, calcium chloride or calcium carbonate. Well-known sugars such as sucrose, glucose, and fructose can be used as appropriate. Meanwhile, a preferred embodiment of the present invention comprises thermally-degradable components with or without sugar or salt. The word thermally-degradable component represents a component which is not thermally-degraded in the kneading step, but is thermally-degraded in the molding step and the sintering step, or is thermally-degraded at a higher temperature than the heating temperature of the molding step or the sintering step. Since the thermally-degradable components are thermally-degraded somewhere in the molding step, the sintering step, or after the sintering step, a bone filling material having appropriate voids can be obtained.

It is preferred that salt or sugar loadings are adjusted as appropriate based on the physical properties required for the bone filling material, but the loadings are, for example, between 1 percent by weight to 20 percent by weight, based on 100 percent by weight of ingredients, or may be 2 percent by weight to 10 percent by weight. The blending ratio of salt or sugar to ingredients is 1 to 30 volume percent, preferably 2 to 20 volume percent, more preferably 3 to 10 volume percent. When thermally-degradable components are added to ingredients, the same amount as salt or sugar is preferred to be added.

In the kneading step, compound which is a material for injection molding is obtained by mixing the above described ingredient powders and binders. If ingredient powders are not uniformly mixed, several problems will be caused. For example, the geometry of a molded body will be deteriorated. In particular, it is preferred that each bone filling material of the present invention have constant geometry in order to maintain the same dosage of pharmaceutical agents. So it is desirable that the ingredients be made as uniform as possible.

The temperature of the kneading step is preferred to be adjusted as appropriate based on the kind of binders. But if the temperature is low, the ingredient powders and the binders can not be mixed, and if the temperature is high, the binders will be thermally degraded. So the temperature is set to be, for example, from 110°C to 240°C, preferably from 130°C to 190°C, and more preferably from 140°C to 160°C.

In the kneading step, it takes long time for kneading ingredients uniformly. But if the kneading time is too long, the binders will be thermally degraded. So the kneading time is preferred to be adjusted as appropriate based on the kinds on binders. The kneading time is, for example, from 30 minutes to 5 hours. It may also be 45 minutes to 1.5 hours.

As a kneading machine used in the kneading step, for example, a pressure type kneader, or a uniaxial or biaxial extrusion kneader can be used as appropriate. Since the bone filling material of the present invention is pharmaceutical agent which will be used for transplant, it is preferred that the bone filling material be free of impurities such as broken pieces of kneading blades of a kneading machine. From this perspective, it is desired that the kneading blades be made of material with high hardness, and kneading blades which are provided by surface protective layers (e.g., deposited by TiN coating) are preferred.

A preferred kneading process is, for example, as follows. Firstly, a kneading machine is heated to a predetermined temperature, and binder having high melting point is cast into the kneading machine. When the binder is dissolved to a certain extent, ingredient powders are cast into the kneading machine. After that, binder having low melting point and ingredient powders are cast into the kneading machine, and 1/2 to 4/5 by volume of ingredient is cast into the kneading machine, followed by casting low-volatile components such as DBP (dibutyl phthalate). And then, the remaining ingredient is cast into the kneading machine. In this way, the aggretation of the ingredient powders could be dispersed by kneading binder having high melting point (high-viscous binder) and ingredient powders in the beginning of the step.

In particular, for example, the kneading step comprises the steps of: putting the (meta) acrylic-based resin and the ethylene-vinyl acetate copolymer in a kneading machine; putting the ingredient, the paraffin wax, and the stearic acid in the kneading machine while kneading the (meta) acrylic-based resin and the ethylene-vinyl acetate copolymer; and putting the dibutyl phthalate in the kneading machine while kneading the (meta) acrylic-based resin, the ethylene-vinyl acetate copolymer, the paraffin wax, and the stearic acid. In this way, the compound which is material for injection molding can be obtained.

However, since the bone filling material of the present invention is replaced with bones in the future, molded products having consciously made minute cracks may be used to promote the replacement. From this perspective, for example, the kneading time may be from 15 minutes to 30 minutes, and the kneading temperature may be from 80°C to 100°C.

The molding step is a process for producing a molded body with a predetermined shape by injection molding. It is preferred that a bone filling agent have four protruding parts extending from the center of the regular tetrahedron form of the bone filling material toward each vertex thereof. Herein after an example of a mold for producing the bone filling material is explained. The above described mold, for example, has: a fixed mold having an inlet (gate) where material is injected; and a movable mold which is contacted with the fixed mold when the material is injected, and is apart from the fixed mold after a molded product is formed.

In the molding step, an injection molding is performed preferably by using an injection molding machine. The injection molding machine is not specifically limited, and a well-known injection molding machine can be used. The examples of the injection molding machine include: a vertical injection molding machine or a horizontal injection molding machine; a high pressure injection molding machine, a moderate pressure injection molding machine, or a low pressure injection molding machine; or a plunger injection molding machine or a screw injection molding machine. However, in order to produce a bone filling material having minute protruding parts from calcium phosphate-based material, an injection molding machine which is a horizontal screw type injection molding machine (which is preferably a high pressure injection molding machine) can be preferably used. However, if impurities such as broken pieces of a screw cylinder are mixed in the bone filling material of the present invention, (although it will not be a problem for an ordinary molded body) it will be a problem because the bone filling material is intended to be administered in vivo. So it is preferred that surface protective layers such as TiN coating layers are preferred to be formed on the surface of the screw.

In the binder removal step, binder contained in the molded body obtained in the above described molded step is removed, and thereby degreased body is produced. The binder removal step is also referred to as a degreasing step. If binder is not removed sufficiently in the binder removal step, the molded body may be cracked or bloated in the sintering step below. In the degreasing step, it is expected that the binder removal is completed without causing defects on the molded body such as deformations and cracks. The examples of binder removal method include the sublimation method, the natural drying method, the solvent extraction method, the thermal degreasing method, and the like, and the thermal degreasing method is preferred. The thermal degreasing method is performed in an ambient atmosphere, a reduced pressure atmosphere, a pressurized atmosphere, a gas atmosphere, or the like, and is preferably performed in an ambient atmosphere. A molded body is preferably placed on a ceramics setter (porous and dense) when it is cast into a degreasing furnace. If the molded body is large (i.e., thick molded body), porous setter such as alumina setter is preferred. It is also desirable to watch for impurities of contaminated setter and components of a heated setter.

A binder removal step, for example, has several stages of heating-up period and duration period in accordance with the pyrolysis temperature of resin contained in binder. In particular, effective pyrolysis of resin having low pyrolysis temperature improves sintering performance. In the present invention, since temperature is raised as above described, resin having low pyrolysis temperature can be effectively pyrolyzed. A preferred embodiment of a bone filling material of the present invention may include compounds having poor biocompatibility in binder, although the bone filling material is administered in vivo. Such compound tends to be a binder having low melting point. So in the heating-up step, in order to vaporize binder having low melting point completely, it is preferred that the temperature be raised relatively moderately. A specific example of heating up ratio is at 1 °C/hour to 3x10² °C/hour until the temperature reaches in the range of 110°C to 300°C which is the temperature of the first maintaining period (preferably until the temperature reaches in the range of 230°C to 250°C), preferably at 1x10 °C/hour to 2x10² °C/hour, more preferably at 2x10 °C/hour to 5x10°C/hour, and the ratio may also be at 3x10 °C/hour to 4x10 °C/hour. The maintaining step is, for example, from 2x 10 minutes to 5 hours, preferably from 3x10 minutes to 2 hours.

The sintering step is a step for heating a molded body obtained in the binder removal step. Japanese Patent Laid-Open No. 2004-97259 (paragraph [0025]) shows a sintering at 1,250 °C for an hour. But in a preferred embodiment of the present invention, a molded body is heated from ambient atmosphere to the highest temperature 9x10²°C to 1.1x10³ °C. This is, for example, to turn α-TCP, as ingredient, effectively into β-TCP. High temperature maintaining period is, for example, 5x10⁻¹ hours to 3 hours. It is noted that in the sintering step, a heating-up step (and maintaining step) is followed by a cooling step, wherein well-known cooling methods are used as appropriate. The sintering period including the cooling period is, for example, from 6 hours to 5x10 hours, preferably from 1x10 hours to 3x10 hours. The molding temperature is, for example, from 1x10² °C to 1.5x10² °C. And the mold temperature is, for example, from 1x10°C to 3x10°C.

The aftertreatment step is an optional step for aftertreatment of the molded body obtained in the sintering step. Specific examples of the aftertreatment step include patching holes caused by ejector pins, and cleaning the molded body.

It is another preferred embodiment to add well-known pharmaceutical agent in addition to ingredient powder. The bone filling material containing the pharmaceutical agent serves as carriers thereof because the volume of the bone filling material produced in the present invention is approximately uniform. It is preferred that the pharmaceutical agent added to the bone filling material remain activity at high temperatures.

A bone filler is also referred to as a bone filling agent, which is filled in a bone deficit site. A bone filler may be retained in vivo, but preferably be merged with bone tissues. As a composition of a specific bone filler, a well-known composition can be used as appropriate. In particular, the composition of a bone filler contains calcium-based material. The example of the calcium-based material is one or both of calcium phosphate-based material or calcium carbonate-based material. Specifically, it includes one or more than one kind of: hydroxyl apatite, carbonate apatite, fluorine apatite, chlorine apatite, β-TCP, α-TCP, calcium metaphosphate, tetracalcium phosphate, calcium hydrogenphosphate, calcium dihydrogen phosphate, calcium pyrophosphate, salts thereof, or solvate thereof.

A preferred embodiment of a bone filler of the present invention contains pharmaceutical agent as appropriate. In particular, in a step for producing a bone filler, pharmaceutical agent is preferred to be added to the main component of a bone filler such as calcium phosphate-based material.

Another preferred embodiment of the present invention is a bone filling material to which a pharmaceutical agent is impregnated or administered as needed. The methods for coating pharmaceutical agent includes impregnation coating, spray coating, and spin-coat coating, wherein pharmaceutical composition which is obtained by dissolving pharmaceutical agent with well-known pharmaceutically acceptable diluent (solvent) is administered. Among them, impregnation coating is preferred. Impregnation coating of pharmaceutical agent impregnates the surface or inside of the bone filling agent with pharmaceutical agent. Namely, the present invention can provide a bone filling material to which a predetermined pharmaceutical agent is impregnated or coated. Note that below explained drug agent may be mixed with material which is used when bone filling material is produced by the PR method and the like.

A preferred embodiment of the bone filling material of the present invention is the above described bone filling material wherein the pharmaceutical agent comprises an osteogenesis/chondrogenesis promoter (including chondrogenesis promoting factor), a joint disease therapeutic agent, a preventive and/or therapeutic agent for bone/cartilage disease, a bone-regenerating agent, a bone resorption-suppressing substance, an angiogenesis promoter, an antibacterial agent, an antibiotics, or an anticancer agent. A preferred embodiment of the bone filling material of the present invention is the above described bone filling material wherein the pharmaceutical agent comprises thienoindazole derivative represented by the below described general formula (I). The thienoindazole derivative (4,5- dihydro -1 - methyl - 1H- thieno [3,4-g] indazole derivative) can be produced in accordance with a method disclosed in the Japanese Patent Laid-Open No. 2002-356419. It is desirable that effective dose of the pharmaceutical agent that provides a predetermined efficacy is contained in the bone filling material of the present invention. Namely, since well-known pharmaceutical agent can be used in the present invention, the amount of pharmaceutical agent contained in the bone filling material is preferred to be adjusted as appropriate so that the effective dose of the pharmaceutical agent that provides a predetermined efficacy can be administered.

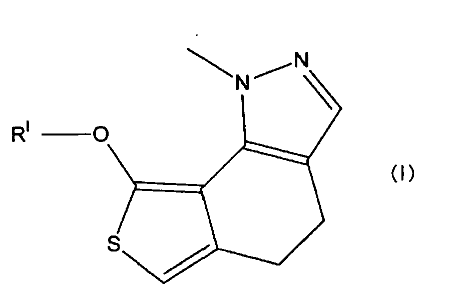

wherein R^{I} represents a carboxyamido group (-CH (NH₂) (CO₂H), -CH (NH₂) (SO₃H), -CH (NH₂) (SO₂NHR^{II}), -CH (NH₂) (PO (NH₂) OH) and -CH (NH₂) (PO (OR^{II}) OH), (where R^{II} is a C₁₋₅ linear alkyl group). Among them, carboxyamido group is the most preferred.

As the osteogenesis/chondrogenesis promoter, a publicly known agent for inducing osteogenesis or chondrogenesis can be used as needed. In particular, a chondrogenesis promoter is, for example, a 2-[1-(2,2- Diethoxy-ethyl) -3-(3-p-tolyl-ureido) -2, 3-dihydro-1H-indol-3-yl] -N-p-tolyl-acetamide which is disclosed in WO 02/087620 pamphlet. As a chondrogenesis promoter, for example, osteogenesis promoting factor can be used. The osteogenesis promoting factor is generally referred to as BMP (bone morphogenetic protein). The BMP is a substance for bone/cartilage induction which acts on undifferentiated mesenchymal cells from outside, differentiating them to chondrocyte or osteoblasts. As the osteogenesis promoting factor, for example, BMP1 to 13 can be used. The BMP used as a pharmaceutical agent of the present invention may be either one of the BMP obtained by genetic recombination or the purified BMP taken form Dunn osteogenic sarcoma (see Takaoka, K., Biomedical Research, 2 (5) 466-471 (1981)).

Examples of the joint disease treating agent include: anti-inflammatory steroid agents such as p38MAP kinase inhibitor (thiazole-based compound etc., disclosed in WO00/64894), matrix metalloprotease inhibitor (MMPI), prednisolone, hydrocortisone, methylpredinisolone, dexabethamethasone, and bethamethasone; and non-steroidal anti-inflammatory analgesic agents such as indometacin, diclofenac, loxoprofen, ibuprofen, piroxicam, and sulindac.

Examples of the bone/cartilage disease preventing or treating agent include one or mixtures of more than one kind of the following substances: non-peptide osteogenesis-promoting substances such as prostaglandin A1 derivative, vitamin D derivative, vitamin K₂ derivative, eicosapentaenoic acid derivative, benzylphosphonic acid, bisphosphonic acid derivative, sex hormone derivative, phenolsulfophthalein derivative, benzothiopyran or benzothiepine derivative, thienoindazole derivative, menatetrenone derivative, helioxanthine derivative; and a hardly soluble peptide osteogenesis-promoting substance. These substances can be obtained by a known method. A bone/cartilage disease preventing agent includes one or both of a cartilage disease preventive agent and an agent preventing the situation that a bone/cartilage disease develops.

Examples of the bone-regenerating agent include one kind or a mixture of more than one kind of the following substances: calmodulin; actinomycin D; cyclosporin A; glucosamine sulfate; glucosamine hydrochloride; marrow extract; calcium phosphate; lactic acid/ glycolic acid/ ε-caprolactone copolymer; platelet rich plasma; or human marrow mesenchymal cell. These substances can be obtained by a known method.

Examples of the bone resorption-suppressing substance include one kind or a mixture of more than one kind of estrogenic agent, calcitonin, and bisphosphonate. These substances can be obtained by a known method.

Examples of the angiogenesis promoter include one kind or a mixture of more than one kind of the following substances: indigocarmine; 4- [N-methyl-N-(2-phenylethyl) amino] -1- (3, 5-dimethyl-4-propionyl aminobenzoyl) piperidine; 4-(5H-7, 8, 9, 10- tetrahydro-5, 7, 7, 10, 10-pentamethyl benzo [e] naphtho [2, 3-b] [1,4] diazepine-13-yl) benzoic acid; activated protein C; urotensin II -like peptide compound; fibroblast growth factor (FGF) (including basic FGF and acidic FGF); vascular endothelial cell growth factors (VEGF) (preferably a platelet-derived factor); hepatocyte growth factor (HGF); angiopoetin (including angiopoetin-1 and angiopoetin-2); platelet-derived growth factor (PDGF), Insulin-like growth factor (IGF) or smooth muscle embryo myosin heavy chain(SMemb). Of these substances, fibroblast growth factor is preferred (see, Hockel, M. et al., Arch. Surg., No. 128, p. 423, 1993). A basic fibroblast growth factor (bFGF) is preferred as a fibroblast growth factor, and a specific example includes trafermin (gene recombinant). Namely, a preferred embodiment of the bone filling material of the present invention is the above described bone filling material comprising trafermin, a salt thereof, a solvate thereof, or a prodrug thereof. "A salt thereof" represents a salt of trafermin, and is specifically the same salt as above explained. "A solvate thereof' represents a solvate of trafermin, and is specifically the same solvate as above explained. "A prodrug thereof" represents a prodrug of trafermin, and represents an agent that turns into, for example, trafermin, an ion thereof, or a salt thereof in vivo after administration. In particular, a prodrug contains protecting groups such as an amino group which are taken off in vivo, and acts the same as trafermin.

As an antibacterial agent or an antibiotic, a well-known antibacterial agent or antibiotic can be used as appropriate. Specific examples of antibacterial agents or antibiotics include one kind or a mixture of more than one kind as appropriate of the following substances: sulfonamide such as sulfacetamide, sufamethizol, sulfadimidine, and sulfamerazine; chloramphenicols antibacterial agent such as chloramphenicol (CP), and tiamphenicol; quinolones antibacterial agent such as ofloxacin (OFLX), ciprofloxacin (CPFX), enrofloxacin, lomefloxacin (LFLX), rufloxacin, levofloxacin (LVFX), fleroxacin (FLRX), nadifloxacin (NDFX), norfloxacin (NFLX), and sparfloxacin (SPFX); fusidic acid (FA); fusafungine; fosfomycin (FOM), mupirocin (MUP); brodimoprim; dirithromycin; penicillins antibacterial agent such as benzylpenicillin (PCG); penicillin G procaine; benzathine penicillin, phenoxymethylpenicillin (Penicillin V), methicillin, ampicillin (ABPC), cloxacillin (MCIPC), carbenicillin, pivampicillin (PVPC), amoxicillin (AMPC), talampicillin (TAPC), bacampicillin (BAPC), ticarcillin (TIPC), azlocillin, mezlocillin, pivmecillinam (PMPC), piperacillin (PIPC), amoxicillin (AMPC) - clavulanic-acid (CVA) (co-amoxiclav), apalcillin, temocillin, ticarcillin-clavulanic acid (CVA), ampicillin (ABPC) - sulbactam (SBT), sultamicillin (SBTPC), and piperacillin (PIPC) - tazobactam (TAZ); streptomycins antibiotics such as streptomycin (SM); tetracyclines antibiotics such as chlortetracycline, aureomycin, chloramphenicol (CP), oxytetracycline (OTC), demethylchlortetracycline, demeclocycline, ledermycin®, lymecycline, doxycycline (DOXY), and minocycline (MINO); aminoglycosides antibiotic such as neomycin, spectinomycin (SPCM), gentamycin (GM), tobramycin (TOB), amikacin (AMK), micronomicin (MCR), isepamicin (ISP), and arbekacin (ABK); macrolides antibiotics such as erythromycin (EM), spiramycin (SPM), roxithromycin (RXM), azithromycin (AZM), midecamycin (MDM), and clarithromycin (CAM); glycopeptides antibiotics such as vancomycin (VCM), and teicoplanin (TEIC); polypeptides antibiotics such as colistin (CL); streptogramins antibiotics such as virginiamycin, and pristinamycin; lincomycins antibiotics such as clindamycin (CLDM); cephalosporins antibiotics such as cephalexin (CEX), cefazolin (CEZ), cefradine (CED), cefadroxil (CDX), cefamandole (CMD), cefuroxime (CXM), cefaclor(CCL), cefotaxime (CTX), cefsulodin (CFS), cefperazone, cefotiam (CTM), ceftriaxone (CTRX), cefmenoxime (CMX), ceftazidime (CAZ), ceftiroxime, cefonicid, cefpiramide (CPM), cefoperazone (CPZ) - sulbactam (SBT), cefpodoxime (CPDX), cefozidime, cefdinir (CFDN), cefetamet (CEMT), cefpirome (CPR), cefprozil, ceftibufen, and cefepime (CFPM); cephamycins antibiotics such as cefoxitin (CFX), cefmetazole (CMZ), and cefotetan (CTT); oxacephems antibiotics such as latamoxef (LMOX), and flomoxef (FMOX); carbapenems antibiotics such as imipenem (IPM) - cilastatin (CS) (tienam®); monobactams antibiotics such as aztreonam (AZT); carbacephems antibiotics such as loracarbef (LCBF); carbapenems antibiotics such as panipenem (PAPM) - betamipron (BP); ketolides antibiotic such as telithromycin (TEL).

Anticancer agent is a pharmaceutical agent for treating or preventing cancer. A publicly known anticancer agent can be used as needed. Specific examples of anticancer agent include the following substances: anticancer hemolytic streptococcus formulation such as OK-432 (commercial name Picibanil); anticancer polysaccharide such as krestin, lentinan, schizophyllan, and sonifilan; anticancer antibiotics such as mitomycin C (commercial name Mitomycin, etc.), actinomycin D (commercial name Cosmegen), bleomycin hydrochloride (commercial name Bleo), bleomycin sulfate (commercial name Bleo S), daunorubicin hydrochloride (commercial name Daunomycin), doxorubicin hydrochloride (commercial name Adriacin), neocarzinostatin (commercial name Neocarzinostatin), aclarubicin hydrochloride (commercial name Aclacinon), or epirubicin hydrochloride (commercial name Farmorubicin); mitotic inhibitor such as Vinblastine; alkylating agent such as cis-platin, carboplatin, and cyclophosphamide; antimetabolite such as 5-fluorouracil, cytosine arabinoside and hydroxyurea, N- {5- [N-(3, 4-dihydro-2-methyl-4-oxoquinazoline-6-ylmethyl) -N-methylamino] -2- thenoyl} -L-glutamic acid; anticancer antibiotics such as adriamycin and bleomycin; enzyme such as asparaginase; topoisomerase inhibitor such as etoposide; biological response modifier such as Interferon; antiestrogen such as "NOLVADEX" (tamoxifen); antiandrogen such as "CASODEX"; antimetabolite such as Fluorouracil, Tegafur, Tegafur-uracil, and Methotrexate; plant alkaloid such as Vncristine; anticancer antibiotic such as mitomycin C, actinomycin D, bleomycin hydrochloride, bleomycin sulfate, daunorubicin hydrochloride, doxorubicin hydrochloride, neocarzinostatin, aclarubicin hydrochloride, Aclacinon, and epirubicin hydrochloride; and platinum complex such as cyclotriphosphazene-platinum complex, and cisplatin-platinum complex.

It is expected that the bone filling material of the present invention promotes its replacement with bone tissues in vivo, so pharmaceutical agents including a specific polypeptide or gene may be administered or impregnated to the bone filling material. The examples of the polypeptide or the gene include a basic fibroblast growth factor (bFGF), a platelet derived growth factor (PDGF), insulin, an insulin-like growth factor (IGF), a hepatocyte growth factor (HGF), a glial cell line-derived neurotrophic factor (GDNF), a neurotrophic factor (NF), hormone, cytokine, a bone morphogenetic factor (BMP), a transforming growth factor (TGF), a vascular endothelial cell growth factor (VEGF). Among them, a growth factor promoting neoangiogenesis and/or osteogenesis is preferred. The examples of the growth factor include a bone morphogenetic factor (BMP), a bone growth factor (BGF), a vascular endothelial cell growth factor (VEGF) and a transforming growth factor (TGF). The specific example is a calponin gene disclosed in the Japanese Patent No. 3713290. The gene is preferred to be contained in the bone filling material as much as the amount which is effective for the gene therapy. It is also preferred that the gene be contained in the bone filling material as it is (naked), in a micelle state, or in the form of a recombinant vector which is transformed into a known vector such as a virus vector. The pharmaceutical agent may be a known genetic antibody.

The gene can be adjusted based on well-known base sequence in accordance with ordinary methods. For example, cDNA of the targeted gene is adjusted in the following method. RNA is extracted from osteoblasts, and primer is produced based on a well-known base sequence, and then cloning by PCR method. Also commercially available genes may be used.

A preferred embodiment of a bone filling material of the present invention is the above described bone filling material including stabilizer. As the stabilizer, a well-known stabilizer used for polymer compound, in particular, pharmaceutically acceptable stabilizing agents can be used as appropriate. The strength of the bone filling material of the present invention is maintained mainly in vivo for extended period. But it is assumed that the bone filling material is decomposed in the early stages due to the existence of enzymes such as protease. So a preferred embodiment of the present invention includes inhibitors such as protease inhibitor. Well-known enzyme inhibitor can be used for the inhibitor as appropriate. The specific examples include one or more than one kind of the following substances: 4-(2- aminoethyl) benzene sulfonyl fluoride, aprotinin, bestain, calpains inhibitor I, calpains inhibitor II, chymostain, 3,4-Dichloroisocoumain, E-64, EDTA, EGTA, lactacystin, leupeptin, MG-115, MG-132, pepstain A, phenylmethyl sulfonyl fluoride, proteasome inhibitor I, p- toluene sulfonyl - L - lysine chloromethylketone, p- toluene sulfonyl - L - phenylalanine chloromethylketone, or tyrosine inhibitor. These protease inhibitors are commercially available, and the inhabitory concentrations thereof are also commonly known. A preferred embodiment of the compound formed by the bone filling material of the present invention maintains the strength for a prolonged period and has sustained drug release. Therefore, the bone filling material of the present invention preferably contains 2 to 100 times of one dosage of the above protease inhibitor, more preferably contains 2 to 50 times thereof. The specific dose level of the protease inhibitor differs based on the kind of the protease inhibitor to be used. The dose preferably contains the amount of protease inhibitor that makes inhibitor's function effective (i.e., the effective dose). In general, the bone filling material (per 1 g) contains 0.1 µg to 0.5 mg of protease inhibitor. The amount included may be 1 µg to 0.1 mg, or may be 10 µg to 0.1 mg. The specific amount of dosage increases in almost proportion to the volume of the site to which the bone filling material is administered.

Another preferred embodiment of the present invention is a bone filling material (or, a sintered body obtained in the sintering process) to which adhesiveness-imparting agents are impregnated or administered as appropriate. When a heat-resistant adhesiveness-imparting agent is used, the adhesiveness-imparting agent may be mixed with ingredient powders so that a bone filling material which is powder blended with the adhesiveness-imparting agent can be obtained (in this case, the adhesiveness-imparting agent exists on the surface of the bone filling material, and when the adhesiveness-imparting agent on the surface is replaced with bone tissues, a new surface having the other adhesiveness-imparting agent appears, thereby maintaining the adhesiveness of the bone filling material). Also, a powdered adhesiveness-imparting agent may be sprayed on the surface of a molded body or a sintered body. Furthermore, adhesiveness-imparting agents may be added to the surface of the bone filling material by powder blending, wherein a plurality of bone filling agents and powdered adhesiveness-imparting agents are mixed together and then agitated as needed. The adhesiveness-imparting agent may be impregnated or administered with the above mentioned pharmaceutical agent, and the adhesiveness-imparting agent alone may be impregnated or administered. The adhesiveness-imparting agent is an agent for raising adhesion property between the bone filling materials, and it is preferred that the adhesiveness-imparting agent alone do not have high adhesiveness but increase adhesiveness when it contacts with cells in vivo. A specific example of the adhesiveness-imparting agent is Thrombin. Thrombin is one of enzymes which promotes blood clot. Thrombin produces fibrin which is a blood clotting substance in vivo. Fibrin produced by thrombin promotes blood clotting. So when thrombin is used as an adhesiveness-imparting agent, the adhesiveness of the bone filling material will be improved, which raises the strength of the bone filling material by fixing the bone filling materials each other firmly. Thrombin can be impregnated or administered with the same amount of the above described pharmaceutical agent and in the same manner thereof.

The term "a salt thereof" represents a salt of the above described compounds, particularly represents pharmaceutically acceptable salts of the above described compounds. The term "pharmaceutically acceptable" in this specification means that something is not deleterious to the recipient thereof. The polyphosphoric acid of the present invention can be made to salt by in an ordinary method. The examples of the salt includes: the alkaline metal salts such as sodium salt, potassium salt, and lithium salt; the alkaline earth metal salts such as calcium salt, and magnesium salt; the metal salts such as aluminum salt, iron salt, zinc salt, copper salt, nickel salt, and cobalt salt; the inorganic salts such as ammonium salt; and the organic amine salts such as t-octyl amine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N-dibenzylethylenediamine salt, chloroprocaine salt, procaine salt, diethanolamine salt, N-benzyl-N-phenethylamine salt, piperazine salt, tetramethylammonium salt, tris (hydroxymethyl) aminomethane salt. Among these salts, as polyphosphoric acid salt, alkaline metal salt is preferred, and sodium salt is more preferred. In this specification, "a salt thereof" may include not only anhydrous salt but also hydrate salt. These salts, for example, are ionized in vivo, and act the same as the above described compounds.

The term "a solvate thereof" represents a solvate of the above described compounds. The solvate herein includes a hydrate. The agent of the present invention may absorb moisture, be attached with absorption water, and be hydrated when it is left in the atmosphere or recrystallized. The solvates thus obtained are also included in "a solvate thereof". These solvates are ionized in vivo, and act the same as the above described compounds.

### 3-4 Method for Using Bone Filler

The bone filler produced in this way is used, for example, for filling bone deficit site in surgical or orthopedic treatment. As shown in Fig. 1, when the bone filler is filled in the bone deficit site, the strength of the site where the bone filler is filled is maintained because the shape of the bone filler of the present invention and that of the bone deficit site are identical to each other. Further, since the bon filler merges with bone tissues quickly, bone tissues are reproduced in a short period. Namely, the present invention can also provide a therapeutic method for providing a bone filler which is produced in the above method to a patient suffering from a bone defect.

The fourth aspect of the present invention relates to a method for producing a bone filler comprising: a bone model producing step for producing a bone model; a figure-forming material setting step for setting figure-forming material on the bone model, the bone model being obtained in the bone model producing step; and a bone filler producing step for producing a bone filler based on the figure-forming material, the figure-forming material being set on the bone model in the figure-forming material setting step. Note that the figure forming material setting step is preferably a step for setting figure forming material so as to correct asymmetry of a bone model by using a bone model obtained in the bone model producing step. In this way, a bone filler which can correct deformation of a bone can be obtained. A preferred embodiment of the fourth aspect of the present invention relates to a method for producing one of the above described bone filler, wherein the bone model produced in the bone model producing step has contour lines or grid patterns drawn on the surface thereof. A preferred embodiment of the fourth aspect of the present invention relates to a method for producing one of the above described bone filler, wherein the figure-forming material used in the figure-forming material filling step differs from the bone model in one of the X-ray permeability, the infrared ray permeability, or the ultraviolet ray permeability. A preferred embodiment of the fourth aspect of the present invention relates to a method for producing one of the above described bone filler, wherein the bone model is a bone model of a patient having bone defect, a patient suffering from bone deformation, or a patient of cosmetic surgery. The bone filler obtained in the method for producing a bone filler of the present invention can effectively be used for a treatment of bone deformation and a cosmetic surgery, because the bone filler can be used for correcting bone deformation or bone structure.

### 5. Method for Producing a Cast

The fifth aspect of the present invention relates to a method for producing a bone filler and a cast comprising: a bone digital information obtaining step for obtaining bone digital information including a cross-sectional view of pluralities of the bones, the bones located at the specific part of the patient, by photographing the specific part of the patient; a bone model producing step for producing a bone model based on the digital information, the bone model being located at the specific part of the patient, the digital information including a cross-sectional view of pluralities of the bones, the digital information being obtained in the digital information of bones obtaining step; a figure-forming material setting step for setting figure-forming material on the bone model, the figure-forming material being used for a bone filler, the bone model produced in the bone model producing step, and for setting figure-forming material on the bone model, the figure-forming material being used for cast forming, the figure-forming material including material which is different from the figure-forming material used for a bone filler; a figure-forming material digital information obtaining step for obtaining digital information of figure-forming material by photographing the bone model, the bone model whereon the figure-forming material is set in the figure-forming material setting step; and a bone filler and a cast producing step for producing a bone filler and a cast based on the digital information of the figure-forming material, the figure-forming material obtained in the figure-forming material digital information obtaining step.

According to the method for producing a bone filler and a cast of this aspect, a preferred bone filler can be obtained, and a cast which can support the bone filler properly can be designed. The figure forming material used for forming a cast contains material which is different from the figure forming material used for a bone filler. So these shapes can be distinguished from each other by photographing them with a CT scan, an MRI, and the like. In particular, a cast is preferred to be produced in the same way as the method for producing a bone filler so far explained. As for material of the cast, well-known material used for producing a cast can be used as appropriate. Also, the cast may be produced from the same materials as that of the bone filler.

The fifth aspect of the present invention relates to a method for producing a cast comprising: a bone and soft tissues digital information obtaining step for obtaining bone digital information including cross-sectional view of pluralities of the bones, the bones located at the specific part of the patient, and digital information on the soft tissues, the soft tissues located around the bone, by photographing the specific part of the patient; a cast producing step for producing a cast of the specific part of the patient based on the digital information, the digital information including a cross-sectional view of pluralities of bones and soft tissues obtained in the bone and soft tissue digital information obtaining step. Since a cast is produced based on the digital information of soft tissues, a custom-made cast having a shape which is suitable for patients can be produced. Note that in the present specification, the soft tissue means relatively soft tissues other than bones, which are, in particular, internal organs, flesh, skin, and the like. As for the method for producing bone filler or a cast of the present invention, a preferred embodiment and composition of the above method for producing bone filler can be adopted as appropriate. The specific example of the method is as follows. A target part which is symmetrical part (e.g., a skull, upper and lower jaws, four limbs, a pelvis, and the like) is observed, and a part having height which is lower than the other symmetrical part by a predetermined threshold value (e.g., 5 mm) is selected. And a simulation is made to set bone filler on the part, then a cast having a shape which covers the area where bone filler is set is designed. This designation can easily be made by programming a computer to operate in the above way. Namely, a computer which is imputed bone information of a target part and information of soft tissues reads out the program stored in a main memory, performs a predetermined operation. In this way, data for designing a cast can be obtained. Note that it may also design a cast having a shape which makes soft tissue parts raised simply by a predetermined value (e.g., 1 cm).

### 6. Appearance Model, and Method for Producing thereof

The sixth aspect of the present invention relates to an appearance model of a specific part of a body whereon contour lines or grid patterns are drawn. Since contour lines or grid patterns are drawn on the surface thereof, a deformation of a specific part can objectively be grasped. In particular, the degree of deformation can be grasped objectively by comparing an appearance model before and after the operation.

The sixth aspect of the present invention relates to the above described appearance model, wherein the appearance model is a reproduction of the surface of a specific part of a patient's body. The specific parts of a patient, for example, are the above described parts. They are, specifically, a face, a head, four limbs, a chest, a lower abdomen, a waist, and the like.

The sixth aspect of the present invention relates to a method for producing an appearance model comprising: a cross-sectional view digital information obtaining step for obtaining digital information on a cross-sectional view of a specific part of a patient, the specific part including a cross-sectional view of pluralities of bones and soft tissues of the specific part of the patient, by photographing the specific part; a drawing information obtaining step for calculating the height of each part of the surface on the specific part from a base level based on the digital information, the digital information including a cross-sectional view of pluralities of bones and soft tissues obtained in the cross-sectional view digital information obtaining step, or a drawing information obtaining step for calculating the distortion on the surface of each part of the surface of the specific part from a base level; an appearance model producing step for producing a surface model on the specific part of the patient by the rapid prototype method, as well as drawing contour lines or grid patterns based on the distortion on the surface or height obtained in the drawing information obtaining step. In this producing method, an appearance model can be properly produced. Note that the specific part of a patient is, for example, a part where bone filler is embedded, such as a deficit site caused by an accident and the like, and a bone deformation site. Since appearance models before and after surgical operation can be obtained, the extent of the external changes before and after surgical operation can be shown. Note that the appearance model can be produced from the same raw materials and in the same way as the above explained bone model. Namely, the equipment and the method for producing a bone model of the present invention can be used for producing an appearance model as appropriate. The above description is applies mutatis mutandis to avoid repetition.

### 7. Methods for Producing Epithesis and a Mold for Producing Epithesis

The seventh aspect of the present invention relates to a method for producing an epithesis comprising: a cross-sectional view digital information obtaining step for obtaining digital information on a cross-sectional view of a specific part, the cross-sectional view including a cross-sectional view of pluralities of bones and soft tissues of the specific part of a patient, by photographing the specific part of the patient, and a three-dimensional digital figure obtaining step for obtaining a three-dimensional digital figure of the specific part based on the digital information, the digital information including the cross-sectional view of pluralities of bones and soft tissues obtained in the cross-sectional view digital information obtaining step, an epithesis figure data obtaining step for obtaining epithesis figure data based on the three-dimensional digital figure of the specific part obtained in the three-dimensional digital figure obtaining step; and an epithesis producing step by the rapid prototype method based on epithesis figure data obtained in the epithesis figure data obtaining step. In this method for producing epithesis, the techniques explained in the above each method for producing epithesis is preferred to be used as appropriate. "Epithesis" means prostheses or prosthesis apparatus, and is an artifact which is mainly fixed on the surface of a body. Epithesis figure data can be obtained based on three-dimensional digital figure of a specific part in the following way. Digital figure of a specific symmetric part such as a face, a jaw, eyes, four limbs, a pelvis, or soft tissues therearound is obtained, and the obtained information of the symmetric part is affine transformed to be symmetrical. The obtained information is overlapped with the digital information of an object site whereon epithesis is to be fixed, and the difference is calculated. This method can be used in the other method of the present specification. Information on the epithesis figure can also be obtained by inputting the epithesis figure by a pointing device and the like based on digital information of a specific part of a patient. In order to produce epithesis figure based on the obtained epithesis figure data, computer assisted figure forming method such as the above explained rapid prototype method is used as appropriate. In particular, epithesis having color information can be produced in the following way. Color information of an object part is obtained together with external part information in advance by an MRI and the like. The external part information and the color information thereof are stored in relation to each other. And when the external part is formed, pigment such as ink is applied on the part based on the color information which is stored in relation to the external part. Epithesis reflecting color information can also be obtained by setting basic color of an epithesis figure produced by the rapid prototype method based on, for example, information in which the color of the external surface of a specific part of a patient is averaged.

A preferred embodiment of the seventh aspect of the present invention relates to a method for producing a mold used for producing an epithesis, the method comprising: a cross-sectional view digital information obtaining step for obtaining digital information on a cross-sectional view of a specific part, the cross-sectional view including a cross-sectional view of pluralities of bones and soft tissues of the specific part of a patient, by photographing the specific part of the patient, and a three-dimensional digital figure obtaining step for obtaining a three-dimensional digital figure of the specific part based on the digital information, the digital information including the cross-sectional view of pluralities of bones and soft tissues obtained in the cross-sectional view digital information obtaining step, an appearance model producing step for producing an appearance model of the specific part based on the three-dimensional digital figure of the specific part obtained in the three-dimensional digital figure obtaining step; a figure forming material setting step for setting figure forming material on the appearance model, the appearance model obtained in the appearance model producing step; a figure forming material digital information obtaining step for obtaining figure forming material digital information by photographing a bone model on which a figure forming material is set in the figure forming material setting step; a mold information obtaining step for obtaining digital data on a mold for producing an epithesis figure based on the digital information of the figure forming material obtained in the figure forming material digital information obtaining step; and a mold producing step for producing a mold obtained in the mold information obtaining step. For example, when a part having symmetrical appearance is partially defected, the defected part can be reshaped based on the remaining part. Therefore, a method for producing a symmetrical epithesis, or a method for producing a mold which is used for producing an epithesis can be provided. In the present invention, shape information of a specific part is obtained by a CT scan and the like, and an epithesis is designed by a computer based on the information. Since impression material is not needed to be put directly on a patient, a method for producing a minimally invasive epithesis, or a method for producing a mold which is used for producing a minimally invasive epithesis can be provided. Note that the mold for producing an epithesis thus obtained, for example, is preferred to be obtained in the following way. Epithesis base material is produced by putting silicon wax into a mold, and a practitioner refines the obtained material and puts colors thereon. Having obtained an epithesis in this way, it is fixed on a body by an operational method which is generally used in orthopedic surgery and the like.

### Example 1

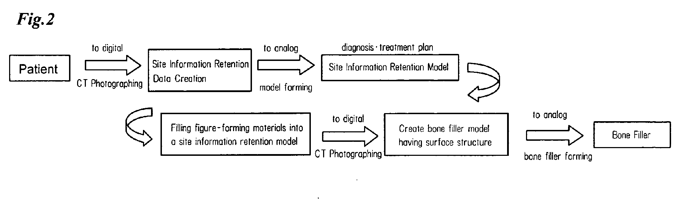

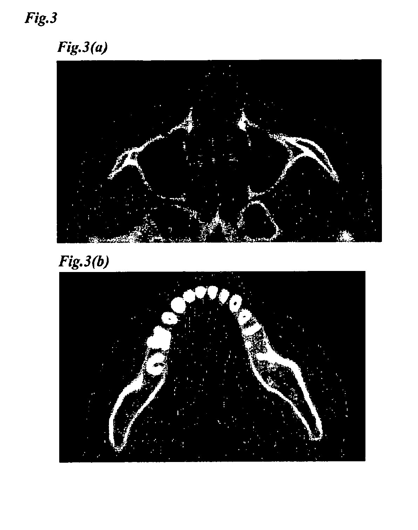

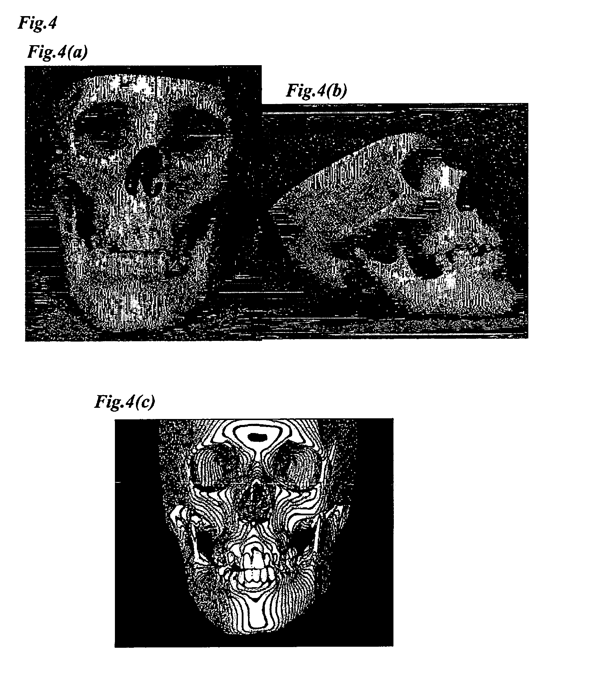

Hereinafter, the present invention is explained specifically by using an example. However, the present invention is not limited to the below example, and the subject matter disclosed in the present specification can be adjusted as appropriate. Fig. 2 is a schematic view showing each step of the present example. In this example, a bone filler which is for correcting bone distortion of a patient suffering from a bone deformation is produced, and the bone distortion is corrected by embedding the bone filler into a patient body. As shown in Fig. 2, in the method for producing a bone filler of this example, a patient's skull (front half portion thereof) is photographed by a CT and dizitized. Fig. 3(a) is a CT image of cheek parts. Fig. 3(b) is a CT image of a lower jaw part. As shown in the CT images of Fig. 3(a) and Fig. 3(b), the bone of the patient was asymmetrically distorted. Based on this CT image, a three-dimensional digital figure of a patient skull was obtained by a computer, and a bone model (1) was formed by the rapid prototype method. Fig. 4(a) shows a gypsum model (a bone model) obtained. Fig. 4(b) shows a side view of a bone model. Fig. 4(c) shows a design drawing of a bone model whereon contour lines are drawn. In this example, a bone model without contour lines (or grid patterns) was used. But the bone model whereon contour lines and the like are drawn as shown in Fig. 4(c) is preferred to be used because bone distortions and the part where bone is formed can easily be grasped.

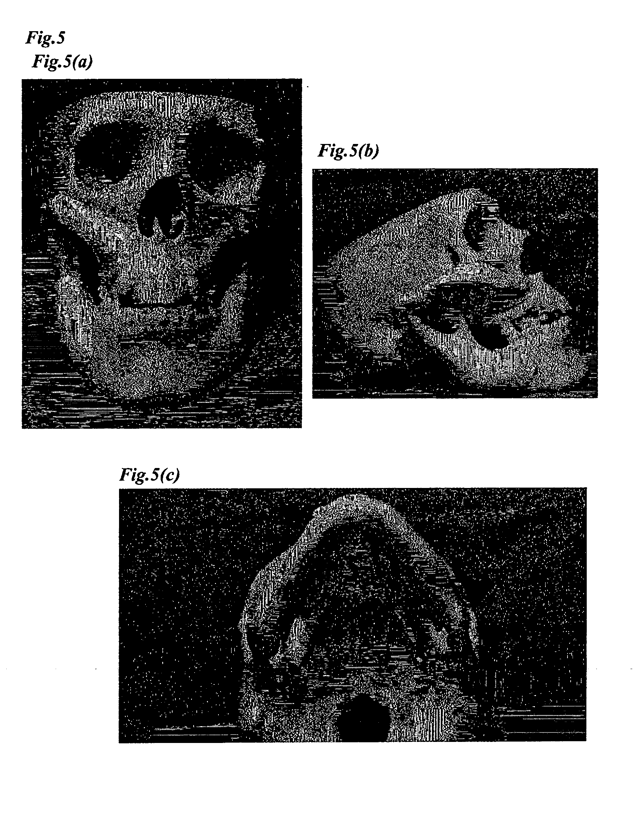

Next, a figure-forming material is set on the bone model in order to correct the distorted parts. In particular, bone fillers are set on dented parts shown in Fig. 3(a), 3(b), and 4(a). Fig. 5(a) is a front view. Fig. 5(b) is a side view. Fig. 5(c) is a bottom view. In this example, the parts whereon figure-forming materials were set can be clearly distinguished from the other parts because the figure-forming material is colored in pink.

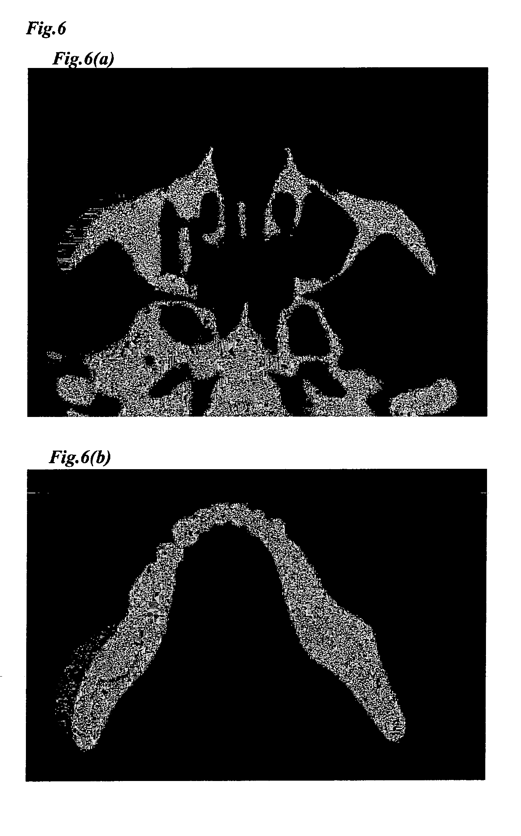

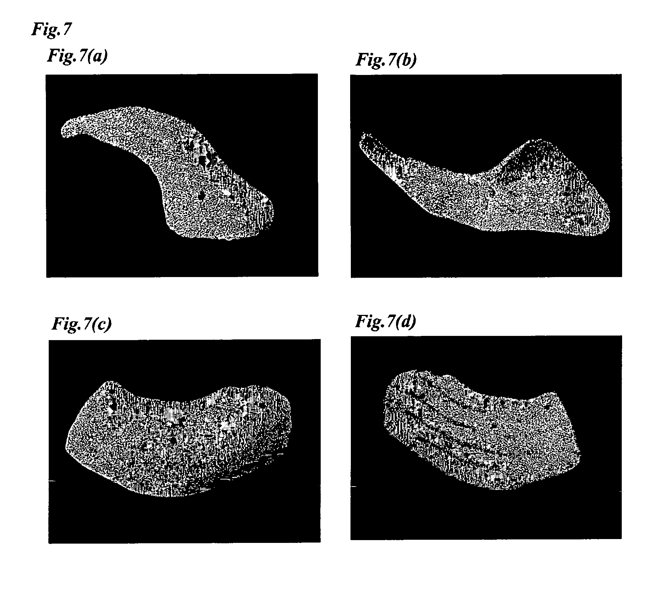

Next, the bone model whereon the figure-forming material is set was imaged by a CT scan. Fig. 6 shows CT images, in place of a diagram, of a bone model whereon figure forming material was set. Fig. 6(a) is a CT image of cheek parts. Fig. 6(b) is a CT image of a lower jaw part. As shown in Fig. 6(a) and Fig. 6(b), the bone model part and the figure-forming material part can clearly be distinguished from each other by CT scanning. And a bone filler was produced by the rapid prototype method based on the obtained three-dimensional digital data of the figure-forming material part. Three holes of diameter ranging from 0.5 mm to 1.5 mm are opened on the obtained bone filler. These holes are used to put threads through the bone model in order to fix the bone model when the bone filler is embedded therein. Fig. 7 shows photographs, in place of a diagram, of a bone filler obtained in the present embodiment. Fig. 7(a) shows a bone filler filled in cheek parts. Fig. 7(b) shows the other side of the bone filler. Fig. 7(c) shows a bone filler filled in a lower jaw part. Fig. 7(d) shows the other side of the bone filler.

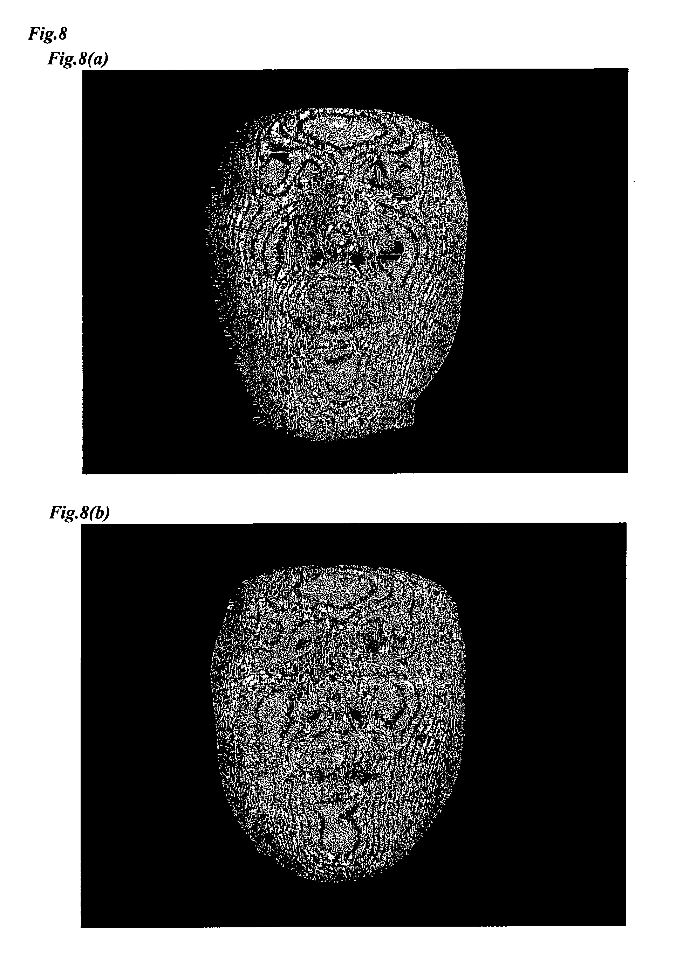

A surgical operation was performed to embed the obtained bone filler into a patient body. The result is shown in Fig. 8. Fig. 8 shows photographs, in place of a diagram, of a gypsum figure (an appearance model) whereon contour lines, which show undulation of a patient's face treated with a bone filler obtained in the example, are drawn. This appearance model was also produced by the rapid prototype method. Fig. 8(a) shows a photograph before the treatment. Fig. 8(b) shows a photograph after the treatment. It can be seen by comparing Fig. 8(b) and Fig. 8(a) that the bone distortion became smaller by the treatment.

The bone model or the appearance model of the present invention can preferably be used in the field of medical equipment industry and the like because surgeons and the like can grasp patient's bone shape easily and precisely.

The method for producing a bone filler of the present invention can preferably be used in the field of medical equipment industry because it can effectively be used for correcting a bone defect, a bone deformation, and the like.

## Claims

1. A method for producing a bone filler comprising:
a digital information of bones obtaining step comprising:
a step of photographing a specific part of a patient; and
a step of obtaining digital information of bones, the digital information including cross-sectional view of pluralities of the bones, the bones located at the specific part of the patient;
a bone model producing step for producing a bone model based on the digital information, the bone model being located at the specific part of the patient, the digital information including cross-sectional view of pluralities of the bones, the digital information being obtained in the digital information of bones obtaining step;
a figure-forming material setting step for setting figure-forming material on the bone model produced in the bone model producing step;
a figure-forming material digital information obtaining step for obtaining digital information of figure-forming material by photographing the bone model, the bone model on which the figure-forming material being set in the figure-forming material setting step; and
a bone filler producing step for producing a bone filler based on the digital information of figure-forming material, the digital information being obtained in the figure-forming material digital information obtaining step.

2. The method for producing a bone filler according to claim 1,
wherein the step of photographing a specific part of a patient is a step for obtaining digital information of a bone including cross-sectional view of pluralities of the bones by a CT scan or an MRI, the bones being located at the specific part of the patient, and
wherein the figure-forming material digital information obtaining step is a step for obtaining the digital information of figure-forming material by a CT scan or an MRI.

3. The method for producing a bone filler according to claim 1,
wherein the specific part of the patient is a site including one of a patient's skull, a lower jaw, an upper jaw, four limbs, or a pelvis.

4. The method for producing a bone filler according to claim 1,
wherein the bone model produced in the bone model producing step has contour lines or grid patterns drawn on the surface thereof.

5. The method for producing a bone filler according to claim 1,
wherein the bone model produced in the bone model producing step is a bone model containing gypsum.

6. The method for producing a bone filler according to claim 1,
wherein the bone model produced in the bone model producing step contains calcium-based material and polyvinyl alcohol resin, and
wherein the polyvinyl alcohol resin is 2 to 8 weight parts when the total weight of the calcium-based material and the polyvinyl alcohol resin is 100 weight parts.

7. The method for producing a bone filler according to claim 1,
wherein the bone model produced in the bone model producing step is made of material containing α-type hemihydrate gypsum and polyvinyl alcohol resin, and
wherein the polyvinyl alcohol resin is 2 to 8 weight parts when the total weight of the calcium-based material and the polyvinyl alcohol resin is 100 weight parts.

8. The method for producing a bone filler according to claim 1,
wherein the bone model producing step is a step for producing a bone model by the rapid prototype method, the injection molding method, the laminate molding method by cutting, or a molding method using processing equipment having a machining center.

9. The method for producing a bone filler according to claim 1,
wherein the bone model contains gypsum as the main ingredient, and
wherein the figure-forming material contains wax or plastic more than 90 weight parts per 100 weight parts of the total amount.

10. The method for producing a bone filler according to claim 1,
wherein the bone model contains gypsum as the main ingredient, and
wherein the figure-forming material contains wax more than 90 weight parts per 100 weight parts of the total amount.

11. The method for producing a bone filler according to claim 1,
wherein the bone model contains gypsum as the main ingredient,
wherein the figure-forming material contains wax more than 90 weight parts per 100 weight parts of the total amount, and
wherein the figure-forming material contains titanium oxide of the rutile type of 2 to 5 weight parts per 100 weight parts of the total amount.

12. The method for producing a bone filler according to claim 1,
wherein the bone filler producing step is a step for producing a bone filler by the rapid prototype method.

13. The method for producing a bone filler according to claim 1,
wherein the bone filler obtained in the bone filler producing step is produced from one or more than one of hydroxyapatite, carbonate apatite, fluorapatite, chlorapatite, β-TCP, α-TCP, calcium metaphosphate, tetra-calcium phosphate, octa-calcium phosphate, calcium hydrogen phosphate, calcium dihydrogen phosphate, calcium pyrophosphate, salts thereof, or solvates thereof.

14. A bone model whereon contour lines or grid patterns are drawn.

15. The bone model according to claim 14,
wherein the bone model is a reproduction of a bone shape of a patient's specific part.

16. The bone model according to claim 14,
wherein the bone model is a reproduction of a bone shape of a patient's skull.

17. A method for producing a bone filler comprising:
a bone model producing step for producing a bone model;
a figure-forming material filling step for filling figure-forming material in a bone defect site of the bone model, the bone model being obtained in the bone model producing step; and
a bone filler producing step for producing a bone filler, the bone filler being filled in the bone defect site based on the figure-forming material, the figure forming material being filled in the bone defect site of the bone model in the figure-forming material filling step.

18. The method for producing a bone filler according to claim 17,
wherein the bone model producing step is a step for producing a bone model by the rapid prototype method.

19. The method for producing a bone filler according to claim 17,
wherein the bone model produced in the bone model producing step has contour lines or grid patterns drawn on the surface thereof.

20. The method for producing a bone filler according to claim 17,
wherein the figure-forming material used in the figure-forming material filling step differs from the bone model in one of the X-ray permeability, the infrared rayspermeability, or the ultraviolet ray permeability.

21. The method for producing a bone filler according to claim 17,
wherein the figure-forming material used in the figure-forming material filling step contains titanium oxide of the rutile type of 2 to 5 weight parts per 100 weight parts of the total amount.

22. The method for producing a bone filler according to claim 17,
wherein the bone model produced in the bone model producing step contains gypsum as the main ingredient,
wherein the figure-forming material used in the figure-forming material filling step contains wax more than 90 weight parts per 100 weight parts of the total amount, and
wherein the figure-forming material contains titanium oxide of the rutile type of 2 to 5 weight parts per 100 weight parts of the total amount.

23. The method for producing a bone filler according to claim 17,
wherein the bone filler producing step is a step for producing a bone filler by the rapid prototype method.

24. The method for producing a bone filler according to claim 17,
wherein the bone filler producing step comprises:
a kneading step for kneading ingredient comprising calcium-based material and material comprising binder;
a molding step for obtaining a molded body having a predetermined shape from a kneaded material with an injection molding machine having a mold, the kneaded material being obtained in the kneading step;
a binder removal step for obtaining a degreased body by removing the binder contained in the molded body obtained in the molding step; and
a sintering step for obtaining a sintered body by heating and sintering the degreased body obtained in the binder removal step.

25. The method for producing a bone filler according to claim 17, further comprising a step of penetrating or administering an osteogenesis/chondrogenesis promoter, a joint disease therapeutic agent, a preventive and/or therapeutic agent for bone/cartilage disease, a bone-regenerating agent, a bone resorption-suppressing substance, an angiogenesis promoter, an antibacterial agent, an antibiotics, or an anticancer agent into the bone filler, the bone filler beeing obtained in the bone filler producing step.

26. A method for producing a bone filler comprising:
a bone model producing step for producing a bone model;
a figure-forming material setting step for setting figure-forming material on the bone model, the bone model being obtained in the bone model producing step; and
a bone filler producing step for producing a bone filler based on the figure-forming material, the figure-forming material being set on the bone model in the figure-forming material setting step.

27. The method for producing a bone filler according to claim 26,
wherein the bone model produced in the bone model producing step has contour lines or grid patterns drawn on the surface thereof.

28. The method for producing a bone filler according to claim 26,
wherein the figure-forming material used in the figure-forming material filling step differs from the bone model in one of the X-ray permeability, the infrared ray permeability, or the ultraviolet ray permeability.

29. The method for producing a bone filler according to claim 26,
wherein the bone model is a bone model of a patient having bone defect, a patient suffering from bone deformation, or a patient of cosmetic surgery.

30. A method for producing a bone filler and a cast, the method comprising:
a bone digital information obtaining step for obtaining bone digital information including a cross-sectional view of pluralities of the bones, the bones located at the specific part of the patient, by photographing the specific part of the patient;
a bone model producing step for producing a bone model based on the digital information, the bone model being located at the specific part of the patient, the digital information including a cross-sectional view of pluralities of the bones, the digital information being obtained in the digital information of bones obtaining step;
a figure-forming material setting step for setting figure-forming material on the bone model, the figure-forming material being used for a bone filler, the bone model produced in the bone model producing step, and for setting figure-forming material on the bone model, the figure-forming material being used for cast forming, the figure-forming material including material which is different from the figure-forming material used for a bone filler;
a figure-forming material digital information obtaining step for obtaining digital information of figure-forming material by photographing the bone model, the bone model whereon the figure-forming material is set in the figure-forming material setting step; and
a bone filler and a cast producing step for producing a bone filler and a cast based on the digital information of the figure-forming material, the figure-forming material obtained in the figure-forming material digital information obtaining step.

31. A method for producing a cast comprising:
a bone and soft tissues digital information obtaining step for obtaining bone digital information including cross-sectional view of pluralities of the bones, the bones located at the specific part of the patient, and digital information on the soft tissues, the soft tissues located around the bone, by photographing the specific part of the patient;
a cast producing step for producing a cast of the specific part of the patient based on the digital information, the digital information including a cross-sectional view of pluralities of bones and soft tissues obtained in the bone and soft tissue digital information obtaining step.

32. An appearance model of a specific part of a body whereon contour lines or grid patterns are drawn.

33. The appearance model according to claim 32,
wherein the appearance model is a reproduction of the surface of a specific part of a patient's body.

34. A method for producing an appearance model comprising:
a cross-sectional view digital information obtaining step for obtaining digital information on a cross-sectional view of a specific part of a patient, the specific part including a cross-sectional view of pluralities of bones and soft tissues of the specific part of the patient, by photographing the specific part;
a drawing information obtaining step for calculating the height of each part of the surface on the specific part from a base level based on the digital information, the digital information including a cross-sectional view of pluralities of bones and soft tissues obtained in the cross-sectional view digital information obtaining step, or a drawing information obtaining step for calculating the distortion on the surface of each part of the surface of the specific part from a base level;
an appearance model producing step for producing a surface model on the specific part of the patient by the rapid prototype method, as well as drawing contour lines or grid patterns based on the distortion on the surface or height obtained in the drawing information obtaining step.

35. A method for producing an epithesis comprising:
a cross-sectional view digital information obtaining step for obtaining digital information on a cross-sectional view of a specific part, the cross-sectional view including a cross-sectional view of pluralities of bones and soft tissues of the specific part of a patient, by photographing the specific part of the patient, and
a three-dimensional digital figure obtaining step for obtaining a three-dimensional digital figure of the specific part based on the digital information, the digital information including the cross-sectional view of pluralities of bones and soft tissues obtained in the cross-sectional view digital information obtaining step,
an epithesis figure data obtaining step for obtaining epithesis figure data based on the three-dimensional digital figure of the specific part obtained in the three-dimensional digital figure obtaining step; and
an epithesis producing step by the rapid prototype method based on epithesis figure data obtained in the epithesis figure data obtaining step.

36. A method for producing a mold used for producing an epithesis, the method comprising:
a cross-sectional view digital information obtaining step for obtaining digital information on a cross-sectional view of a specific part, the cross-sectional view including a cross-sectional view of pluralities of bones and soft tissues of the specific part of a patient, by photographing the specific part of the patient, and
a three-dimensional digital figure obtaining step for obtaining a three-dimensional digital figure of the specific part based on the digital information, the digital information including the cross-sectional view of pluralities of bones and soft tissues obtained in the cross-sectional view digital information obtaining step,
an appearance model producing step for producing an appearance model of the specific part based on the three-dimensional digital figure of the specific part obtained in the three-dimensional digital figure obtaining step;
a figure forming material setting step for setting figure forming material on the appearance model, the appearance model obtained in the appearance model producing step;
a figure forming material digital information obtaining step for obtaining figure forming material digital information by photographing a bone model on which a figure forming material is set in the figure forming material setting step;
a mold information obtaining step for obtaining digital data on a mold for producing an epithesis figure based on the digital information of the figure forming material obtained in the figure forming material digital information obtaining step; and
a mold producing step for producing a mold obtained in the mold information obtaining step.
